(19) Europäisches Patentamt
European Patent Office
Office européen des brevets

(11) EP 2 303 296 B1

(12) EUROPEAN PATENT SPECIFICATION

(45) Date of publication and mention of the grant of the patent:
**20.11.2019 Bulletin 2019/47**

(21) Application number: **09814927.1**

(22) Date of filing: **27.05.2009**

(51) Int Cl.:
*A61K 36/23* (2006.01) *A61K 36/81* (2006.01)
*C12N 15/82* (2006.01)

(86) International application number:
**PCT/US2009/045215**

(87) International publication number:
**WO 2010/033275 (25.03.2010 Gazette 2010/12)**

# (54) ORALLY ADMINISTERABLE VACCINE FOR YERSINIA PESTIS

ORAL VERABREICHBARER IMPFSTOFF FÜR YERSINIA PESTIS

VACCIN CONTRE YERSINIA PESTIS POUVANT ÊTRE ADMINISTRÉ PAR VOIE ORALE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **27.05.2008 US 56365 P**

(43) Date of publication of application:
**06.04.2011 Bulletin 2011/14**

(73) Proprietor: **The Trustees of the University of Pennsylvania**
**Philadelphia, PA 19104-6283 (US)**

(72) Inventor: **DANIELL, Henry**
**Winter Park**
**FL 32792 (US)**

(74) Representative: **Germinario, Claudio et al**
**Società Italiana Brevetti S.p.A.**
**Piazza di Pietra, 38-39**
**00186 Roma (IT)**

(56) References cited:

| | |
|---|---|
| **WO-A1-01/64850** | **WO-A2-01/72959** |
| **WO-A2-2004/005467** | **WO-A2-2007/053183** |
| **US-A1- 2005 108 792** | **US-B1- 6 642 053** |

- **ARLEN PHILIP A ET AL: "Effective plague vaccination via oral delivery of plant cells expressing F1-V antigens in chloroplasts", INFECTION AND IMMUNITY, vol. 76, no. 8, August 2008 (2008-08), pages 3640-3650, XP002687491, ISSN: 0019-9567**
- **ALVAREZ M L ET AL: "Plant-made subunit vaccine against pneumonic and bubonic plague is orally immunogenic in mice", VACCINE, ELSEVIER LTD, GB, vol. 24, no. 14, 24 March 2006 (2006-03-24), pages 2477-2490, XP028010560, ISSN: 0264-410X, DOI: 10.1016/J.VACCINE.2005.12.057 [retrieved on 2006-03-24]**
- **DANIELL H ET AL: "Breakthrough in chloroplast genetic engineering of agronomically important crops", TRENDS IN BIOTECHNOLOGY, ELSEVIER PUBLICATIONS, CAMBRIDGE, GB, vol. 23, no. 5, 1 May 2005 (2005-05-01), pages 238-245, XP027778107, ISSN: 0167-7799 [retrieved on 2005-05-01]**
- **DANIELL H: "Production of biopharmaceuticals and vaccines in plants via the chloroplast genome", BIOTECHNOLOGY JOURNAL, WILEY-VCH VERLAG, WEINHEIM, DE, vol. 1, no. 10, 1 October 2006 (2006-10-01), pages 1071-1079, XP008121149, ISSN: 1860-6768, DOI: 10.1002/BIOT.200600145 [retrieved on 2006-09-27]**
- **HIROSUKE KANAMOTO ET AL: "Efficient and Stable Transformation of Lactuca sativa L. cv. Cisco (lettuce) Plastids", TRANSGENIC RESEARCH, KLUWER ACADEMIC PUBLISHERS-PLENUM PUBLISHERS, NE, vol. 15, no. 2, 1 April 2006 (2006-04-01), pages 205-217, XP019269520, ISSN: 1573-9368**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SHASHI KUMAR ET AL: "Stable transformation of the cotton plastid genome and maternal inheritance of transgenes", PLANT MOLECULAR BIOLOGY, KLUWER ACADEMIC PUBLISHERS, DORDRECHT, NL, vol. 56, no. 2, 1 September 2004 (2004-09-01), pages 203-216, XP019262563, ISSN: 1573-5028, DOI: 10.1007/S11103-004-2907-Y
- CILIA L C LELIVELT ET AL: "Stable Plastid Transformation in Lettuce (Lactuca sativa L.)", PLANT MOLECULAR BIOLOGY, KLUWER ACADEMIC PUBLISHERS, DORDRECHT, NL, vol. 58, no. 6, 1 August 2005 (2005-08-01) , pages 763-774, XP019262736, ISSN: 1573-5028, DOI: 10.1007/S11103-005-7704-8

SEQUENCE LISTING

<110> UNIVERSITY OF CENTRAL FLORIDA RESEARCH FOUNDATION, INC.

<120> ORALLY ADMINISTERABLE VACCINE FOR YERSINIA PESTIS

<130> 10669-045PCT

<140> PCT/US2009/045215
<141> 2009-05-27

<150> 61/056,365
<151> 2008-05-27

<160> 10

<170> PatentIn version 3.5

<210> 1
<211> 513
<212> DNA
<213> Yersinia pestis

<400> 1

```
ttattggtta gatacggtta cggttacagc atcagtgtat ttacctgctg caagtttacc      60
gcctttggaa ccaattgagc gaacaaagaa atcctggctg cccgtagcca agacgacgtc     120
atcccccaca aggttctcac cgtttacctt aggagagata tcaaaatctc tagaatcctt     180
gccaatcact tttgtagtga attggtggtt atttccatcc tgagaagtaa atgttaagta     240
catgggatca cccgcggcat ctgtaaagtt aacagatgtg ctagtggttc ctgttttata     300
gccgccaaga gtaagcgtac caacaagtaa ttctgtatcg atgtttccat tgtccataat     360
tgtaattgga gagccttcct tatatgtaag agtgatgcgg gctggttcaa caagagttgc     420
cgttgcagtg gtgcttgcag ttaaatctgc cgcattagca gttgcaatag ttccaaataa     480
tgcaatggcg ataacggaac tgattttttt cat                                  513
```

<210> 2
<211> 170
<212> PRT
<213> Yersinia pestis

<400> 2

```
Met Lys Lys Ile Ser Ser Val Ile Ala Ile Ala Leu Phe Gly Thr Ile
1               5                   10                  15

Ala Thr Ala Asn Ala Ala Asp Leu Thr Ala Ser Thr Thr Ala Thr Ala
            20                  25                  30
```

```
Thr Leu Val Glu Pro Ala Arg Ile Thr Leu Thr Tyr Lys Glu Gly Ser
        35                  40                  45

Pro Ile Thr Ile Met Asp Asn Gly Asn Ile Asp Thr Glu Leu Leu Val
    50                  55                  60

Gly Thr Leu Thr Leu Gly Gly Tyr Lys Thr Gly Thr Thr Ser Thr Ser
65                  70                  75                  80

Val Asn Phe Thr Asp Ala Ala Gly Asp Pro Met Tyr Leu Thr Phe Thr
                85                  90                  95

Ser Gln Asp Gly Asn Asn His Gln Phe Thr Thr Lys Val Ile Gly Lys
            100                 105                 110

Asp Ser Arg Asp Phe Asp Ile Ser Pro Lys Val Asn Gly Glu Asn Leu
        115                 120                 125

Val Gly Asp Asp Val Val Leu Ala Thr Gly Ser Gln Asp Phe Phe Val
    130                 135                 140

Arg Ser Ile Gly Ser Lys Gly Gly Lys Leu Ala Ala Gly Lys Tyr Thr
145                 150                 155                 160

Asp Ala Val Thr Val Thr Val Ser Asn Gln
                165                 170
```

<210> 3
<211> 2502
<212> DNA
<213> Yersinia pestis

<400> 3

```
tcagttattt aagatgcagg ttgtggataa cctataagca ccagaaatat ccgtttttc      60
tggtagaact acattagatg aacatgattg atttttatct ctcccccact taacaagtat     120
ttttgatttt ttaggtagtc cagtcaaata gacaccgcta ttatctccga caatgccaga     180
gctggatgat tgcccttcaa tggtaactat agaaccaaat ggaacaggtt tattgtcaga     240
gcgttttaaa tgtaaaaaca atcgtccacc aatccttgcg ttaaattttg ctaatactac     300
agctcccttt gtcgggatca cgctaacaat attatttgtt atctctgcat cattgggtaa     360
agtaactggg ttaatttcta ccttattctc tctgtaaggg gttaagtaac cataattggt     420
gtacccccta aagtctgttt tcatgcctgg ccagtatccc actgaagcac cgcttatatc     480
```

aggggcttgt actaatgcaa tagtatctcc agttttttgg ccagccgtta taccgtactg 540
agttattacc atattgccat ttacacctat accaagttgg ctttgggttt gatcgtagct 600
gtagttacca ctgatctccc cataagttcc tcgataattc aaattcagtg cattggaggt 660
atattttctg ccctttttcat taaaacgttc gcgaacgtcc cagtataatt ggcgatcaaa 720
ggcttcaccg tacacaccta cctcatgggt agtgttacca tgagaatctg atgtcatttg 780
gtaatttgaa tttattgagt tattacctag ccagcgactg agaggaaaac tcaaccagat 840
attagataat agctcactag tttttctcc atacttattg atattctgtg tcttcgataa 900
atttaaagtc aatgacatac cattcctaaa aaaatggtta tagcctacag aaaaagaagt 960
tgtgctccta ctgtcacgcc aatagttttt tctgtagcca ctgaaattaa gcgtccccga 1020
accaggtatg ctttgactta aattgaattg cactttgttt ttgggtttag cataatcaaa 1080
acggcaatcg tttctagtat taggtttaca ataagtattt aacgtgtcag cgagtttgtt 1140
aaatccttct gtggcgtatt cctcgctagc aatgtttaac gatgttccac tctgcaagta 1200
cttattatat cgaacgcgcc aacgttggcc gctttctttt ttttgtttat ttttctggct 1260
gtctgcttga gtaacatctg tagatatggc accaaaatca cccaacatag caccgatacc 1320
cagagctgcg gcatgataat tttgggaccc ttgtagtcca ccatataacg taagattcct 1380
tggcaaacca tatttcatcc caagagcgcc aacatacgat gtttgtgtaa gatcattagc 1440
tggacgatat tctcccccca tcattgaata ttcgaaatag cccttccgta atgccactgc 1500
tggtgtgtca tatggaactg taaaaacttg ctttgttcca tcactttcat gaatgatgac 1560
tttcagctca ccactccccc cacccagagg aagatttgct aactcaaatg gtcccgaggg 1620
caccaactca ttacttacag tgtagccatc tcttaaaacc tctaccctgg cttgtgtacg 1680
tgcgataccg cgaacaactg gagcaaaatt ccattggtaa taaggaacca tcgattcatc 1740
tgaagcaatt tttatcccct taatcgggat actgtcaaag atactgctat cagaataggt 1800
ttcccccaat gtcaaacggc tcttaattgt attcaatcct cgctcggcat aaatatacga 1860
acgctgccat ccttgttgtt tccaccatga ggttgaacta cgaaagcgcc aagcccctat 1920
gtttaatccc ggttgcaact gagcataata agagtccaga gacttgcctc cttctctgaa 1980
ttttcttgtc tgcatgttcg tattataatt catgaacaga gcaggaatgc cgtcatccca 2040
caattgcatt ggcataatgc catcaaatct aggtaaaagt gcctgtggtg gcacaattaa 2100
cgataatttc tgctgattaa aataaaactg cacatctgaa tgtggtattg ctaataaatc 2160
aacacattgc tctgtaccag attttattaa atcaggatat ttatctatat caatgccata 2220

```
ctttgtcaat tgtaaggatg ataggcatgg ccaaagaagt tcttttccat tatgtttttc     2280
tagacggaag tcgatatttc cagagtctac ctttcgacca tttacaaaaa cattaacaaa     2340
ataattacct ggaagttgaa gtccttgatt aaaaagagat acatctatac tctctccact     2400
attcgtatca agcatagtag agtcaaaagt atatgcgcgt ccccaacaag gcaatgttgc     2460
caaagttaac cctgcacaca ggaacagctt tgaatacctc at                        2502
```

<210> 4
<211> 775
<212> PRT
<213> Yersinia pestis

<400> 4

```
Met Arg Tyr Ser Lys Leu Phe Leu Cys Ala Gly Leu Thr Leu Ala Thr
1               5                   10                  15

Leu Pro Cys Trp Gly Arg Ala Tyr Thr Phe Asp Ser Thr Met Leu Asp
            20                  25                  30

Thr Asn Ser Gly Glu Ser Ile Asp Val Ser Leu Phe Pro Asp Leu Ile
        35                  40                  45

Lys Ser Gly Thr Glu Gln Cys Val Asp Leu Leu Ala Ile Pro His Ser
    50                  55                  60

Asp Val Gln Phe Tyr Phe Asn Gln Gln Lys Leu Ser Leu Ile Val Pro
65                  70                  75                  80

Pro Gln Ala Leu Leu Pro Arg Phe Asp Gly Ile Met Pro Met Gln Leu
                85                  90                  95

Trp Asp Asp Gly Ile Pro Ala Leu Phe Met Asn Tyr Asn Thr Asn Met
            100                 105                 110

Gln Thr Arg Lys Phe Arg Glu Gly Gly Lys Ser Leu Asp Ser Tyr Tyr
        115                 120                 125

Ala Gln Leu Gln Pro Gly Leu Asn Ile Gly Ala Trp Arg Phe Arg Ser
    130                 135                 140

Ser Thr Ser Trp Trp Lys Gln Gln Gly Trp Gln Arg Ser Tyr Ile Tyr
145                 150                 155                 160
```

```
Ala Glu Arg Gly Leu Asn Thr Ile Lys Ser Arg Leu Thr Leu Gly Glu
                165                 170                 175

Thr Tyr Ser Asp Ser Ser Ile Phe Asp Ser Ile Pro Ile Lys Gly Ile
            180                 185                 190

Lys Ile Ala Ser Asp Glu Ser Met Val Pro Tyr Tyr Gln Trp Asn Phe
        195                 200                 205

Ala Pro Val Val Arg Gly Ile Ala Arg Thr Gln Ala Arg Val Glu Val
    210                 215                 220

Leu Arg Asp Gly Tyr Thr Val Ser Asn Glu Leu Val Pro Ser Gly Pro
225                 230                 235                 240

Phe Glu Leu Ala Asn Leu Pro Leu Gly Gly Gly Ser Gly Glu Leu Lys
                245                 250                 255

Val Ile Ile His Glu Ser Asp Gly Thr Lys Gln Val Phe Thr Val Pro
            260                 265                 270

Tyr Asp Thr Pro Ala Val Ala Leu Arg Lys Gly Tyr Phe Glu Tyr Ser
        275                 280                 285

Met Met Gly Gly Glu Tyr Arg Pro Ala Asn Asp Leu Thr Gln Thr Ser
    290                 295                 300

Tyr Val Gly Ala Leu Gly Met Lys Tyr Gly Leu Pro Arg Asn Leu Thr
305                 310                 315                 320

Leu Tyr Gly Gly Leu Gln Gly Ser Gln Asn Tyr His Ala Ala Ala Leu
                325                 330                 335

Gly Ile Gly Ala Met Leu Gly Asp Phe Gly Ala Ile Ser Thr Asp Val
            340                 345                 350

Thr Gln Ala Asp Ser Gln Lys Asn Lys Gln Lys Lys Glu Ser Gly Gln
        355                 360                 365

Arg Trp Arg Val Arg Tyr Asn Lys Tyr Leu Gln Ser Gly Thr Ser Leu
    370                 375                 380

Asn Ile Ala Ser Glu Glu Tyr Ala Thr Glu Gly Phe Asn Lys Leu Ala
385                 390                 395                 400
```

Asp Thr Leu Asn Thr Tyr Cys Lys Pro Asn Thr Arg Asn Asp Cys Arg
405 410 415

Phe Asp Tyr Ala Lys Pro Lys Asn Lys Val Gln Phe Asn Leu Ser Gln
420 425 430

Ser Ile Pro Gly Ser Gly Thr Leu Asn Phe Ser Gly Tyr Arg Lys Asn
435 440 445

Tyr Trp Arg Asp Ser Arg Ser Thr Thr Ser Phe Ser Val Gly Tyr Asn
450 455 460

His Phe Phe Arg Asn Gly Met Ser Leu Thr Leu Asn Leu Ser Lys Thr
465 470 475 480

Gln Asn Ile Asn Lys Tyr Gly Glu Lys Thr Ser Glu Leu Leu Ser Asn
485 490 495

Ile Trp Leu Ser Phe Pro Leu Ser Arg Trp Leu Gly Asn Asn Ser Ile
500 505 510

Asn Ser Asn Tyr Gln Met Thr Ser Asp Ser His Gly Asn Thr Thr His
515 520 525

Glu Val Gly Val Tyr Gly Glu Ala Phe Asp Arg Gln Leu Tyr Trp Asp
530 535 540

Val Arg Glu Arg Phe Asn Glu Lys Gly Arg Lys Tyr Thr Ser Asn Ala
545 550 555 560

Leu Asn Leu Asn Tyr Arg Gly Thr Tyr Gly Glu Ile Ser Gly Asn Tyr
565 570 575

Ser Tyr Asp Gln Thr Gln Ser Gln Leu Gly Ile Gly Val Asn Gly Asn
580 585 590

Met Val Ile Thr Gln Tyr Gly Ile Thr Ala Gly Gln Lys Thr Gly Asp
595 600 605

Thr Ile Ala Leu Val Gln Ala Pro Asp Ile Ser Gly Ala Ser Val Gly
610 615 620

Tyr Trp Pro Gly Met Lys Thr Asp Phe Arg Gly Tyr Thr Asn Tyr Gly
625 630 635 640

Tyr Leu Thr Pro Tyr Arg Glu Asn Lys Val Glu Ile Asn Pro Val Thr
645 650 655

Leu Pro Asn Asp Ala Glu Ile Thr Asn Asn Ile Val Ser Val Ile Pro
660 665 670

Thr Lys Gly Ala Val Val Leu Ala Lys Phe Asn Ala Arg Ile Gly Gly
675 680 685

Arg Leu Phe Leu His Leu Lys Arg Ser Asp Asn Lys Pro Val Pro Phe
690 695 700

Gly Ser Ile Val Thr Ile Glu Gly Gln Ser Ser Ser Ser Gly Ile Val
705 710 715 720

Gly Asp Asn Ser Gly Val Tyr Leu Thr Gly Leu Pro Lys Lys Ser Lys
725 730 735

Ile Leu Val Lys Trp Gly Arg Asp Lys Asn Gln Ser Cys Ser Ser Asn
740 745 750

Val Val Leu Pro Glu Lys Thr Asp Ile Ser Gly Ala Tyr Arg Leu Ser
755 760 765

Thr Thr Cys Ile Leu Asn Asn
770 775

<210> 5
<211> 810
<212> DNA
<213> Yersinia pestis

<400> 5

tcataaagtc acatttttgg aatacaaacg atccaaccct ccctgatcat taattattct 60

ccacgaaaca ttacgtgctc ccgctagtcc ttttggcaaa tcaaaagccc acgtcgattt 120

aggtggaata tagtgagaag gaatactttt ccctccaaat gttaattcac ctatgttcat 180

gtagaaaggt gaggggtttt cagcaattag cttcccccca tcaactttcc agcttaagtt 240

ttcagcaaac tgtatagggg ttccttttaa ttcattcggt cgaaccaaaa gcttaatgca 300

attattaatt gcgaattgca cgaacactcc cacatctttg tctggattga atttttgctt 360

```
atttgtcgca tcatcaaccc atatatcttc atcctttggt ggaatccctt ttacgcataa      420
ccactttagg ctctctttat ctcgcgggaa aacacctcca gcctgagcta tacgcaaaga      480
attttgttgc ttagcatcca atcgaaacaa tggcggagtg accacgaaag gatcctctga      540
ttctttttct ttattctcgt cgtagatcct agactgaatg agaaccggat aatcttgggt      600
gtttttcacc gagaccataa cgccagcagc atctaacggg tatatgatcc tactctcacc      660
tatagtcacg ccatactctt tgcttgcgaa tttgatatct ggttgagcag agttcgcagc      720
aaaactaagc atgccgaaag taataattcc taacgtactt aatctattta aaatcatgag      780
cttaacctcc ttacggaatg gtgacaacac                                        810
```

<210> 6
<211> 269
<212> PRT
<213> Yersinia pestis

<400> 6

```
Met Leu Ser Pro Phe Arg Lys Glu Val Lys Leu Met Ile Leu Asn Arg
1               5                   10                  15

Leu Ser Thr Leu Gly Ile Ile Thr Phe Gly Met Leu Ser Phe Ala Ala
            20                  25                  30

Asn Ser Ala Gln Pro Asp Ile Lys Phe Ala Ser Lys Glu Tyr Gly Val
        35                  40                  45

Thr Ile Gly Glu Ser Arg Ile Ile Tyr Pro Leu Asp Ala Ala Gly Val
    50                  55                  60

Met Val Ser Val Lys Asn Thr Gln Asp Tyr Pro Val Leu Ile Gln Ser
65                  70                  75                  80

Arg Ile Tyr Asp Glu Asn Lys Glu Lys Glu Ser Glu Asp Pro Phe Val
                85                  90                  95

Val Thr Pro Pro Leu Phe Arg Leu Asp Ala Lys Gln Gln Asn Ser Leu
            100                 105                 110

Arg Ile Ala Gln Ala Gly Gly Val Phe Pro Arg Asp Lys Glu Ser Leu
        115                 120                 125

Lys Trp Leu Cys Val Lys Gly Ile Pro Pro Lys Asp Glu Asp Ile Trp
    130                 135                 140
```

```
Val Asp Asp Ala Thr Asn Lys Gln Lys Phe Asn Pro Asp Lys Asp Val
145                 150                 155                 160

Gly Val Phe Val Gln Phe Ala Ile Asn Asn Cys Ile Lys Leu Leu Val
                165                 170                 175

Arg Pro Asn Glu Leu Lys Gly Thr Pro Ile Gln Phe Ala Glu Asn Leu
            180                 185                 190

Ser Trp Lys Val Asp Gly Gly Lys Leu Ile Ala Glu Asn Pro Ser Pro
        195                 200                 205

Phe Tyr Met Asn Ile Gly Glu Leu Thr Phe Gly Gly Lys Ser Ile Pro
    210                 215                 220

Ser His Tyr Ile Pro Pro Lys Ser Thr Trp Ala Phe Asp Leu Pro Lys
225                 230                 235                 240

Gly Leu Ala Gly Ala Arg Asn Val Ser Trp Arg Ile Ile Asn Asp Gln
                245                 250                 255

Gly Gly Leu Asp Arg Leu Tyr Ser Lys Asn Val Thr Leu
            260                 265
```

<210> 7
<211> 924
<212> DNA
<213> Yersinia pestis

<400> 7

```
ttgatttggg ttatattcat gctaaaacag atgactgtaa attcaattat tcaatatata      60
gaagagaatc tcgagtcgaa attcattaac attgactgtt tggttttgta ttcaggattc     120
agcagaaggt atttgcaaat ttcctttaag gaatatgtcg gaatgcctat tggaacatat     180
attagagtta gaagggctag tagagctgct gcactattac ggcttaccag gctgacaata     240
atagagatat cagcaaagct tttttatgat tcgcaacaga cattcaccag agaatttaag     300
aaaatatttg gttatacccc acggcagtat aggatgatcc ctttttggtc ctttaaaggt     360
ttgttgggta gaagggaaat taactgtgaa taccttcaac cacgaatctg ttaccttaaa     420
gagagaaata taattggtca atgctttaat tttagggatt tagtgttcta ctctgggata     480
gattcaaaat gtagattggg taagttatat gattcgttga agaaaaatac agctataaca     540
gtatcaaaca gaatcccctt tcatgataaa acgaatgaca ttattgcaag aacggttgtt     600
```

```
tgggatagga ataagcattt cagcgatagt gaaataaagg tagataaagg cctgtatgct      660
tatttttttct tcaatgatac atatgatcag tatgttcatc acatgtacaa catatattat     720
aactctttgc ctatttataa tttaaataag cgggatggtt acgatgtgga ggtcataaaa      780
agacgaaatg acaatactat tgattgtcat tattttctcc cgatttattg tgatgacatg      840
gagttttaca atgaaatgca ggtatatcac aataatattg tgaagccgga aatgtcagta      900
acattaggat taccaaagag ttaa                                             924
```

<210> 8
<211> 307
<212> PRT
<213> Yersinia pestis

<400> 8

```
Met Ile Trp Val Ile Phe Met Leu Lys Gln Met Thr Val Asn Ser Ile
1               5                   10                  15

Ile Gln Tyr Ile Glu Glu Asn Leu Glu Ser Lys Phe Ile Asn Ile Asp
            20                  25                  30

Cys Leu Val Leu Tyr Ser Gly Phe Ser Arg Arg Tyr Leu Gln Ile Ser
        35                  40                  45

Phe Lys Glu Tyr Val Gly Met Pro Ile Gly Thr Tyr Ile Arg Val Arg
    50                  55                  60

Arg Ala Ser Arg Ala Ala Ala Leu Leu Arg Leu Thr Arg Leu Thr Ile
65                  70                  75                  80

Ile Glu Ile Ser Ala Lys Leu Phe Tyr Asp Ser Gln Gln Thr Phe Thr
                85                  90                  95

Arg Glu Phe Lys Lys Ile Phe Gly Tyr Thr Pro Arg Gln Tyr Arg Met
            100                 105                 110

Ile Pro Phe Trp Ser Phe Lys Gly Leu Leu Gly Arg Arg Glu Ile Asn
        115                 120                 125

Cys Glu Tyr Leu Gln Pro Arg Ile Cys Tyr Leu Lys Glu Arg Asn Ile
    130                 135                 140

Ile Gly Gln Cys Phe Asn Phe Arg Asp Leu Val Phe Tyr Ser Gly Ile
145                 150                 155                 160
```

```
Asp Ser Lys Cys Arg Leu Gly Lys Leu Tyr Asp Ser Leu Lys Lys Asn
                165                 170                 175

Thr Ala Ile Thr Val Ser Asn Arg Ile Pro Phe His Asp Lys Thr Asn
            180                 185                 190

Asp Ile Ile Ala Arg Thr Val Val Trp Asp Arg Asn Lys His Phe Ser
        195                 200                 205

Asp Ser Glu Ile Lys Val Asp Lys Gly Leu Tyr Ala Tyr Phe Phe Phe
    210                 215                 220

Asn Asp Thr Tyr Asp Gln Tyr Val His His Met Tyr Asn Ile Tyr Tyr
225                 230                 235                 240

Asn Ser Leu Pro Ile Tyr Asn Leu Asn Lys Arg Asp Gly Tyr Asp Val
                245                 250                 255

Glu Val Ile Lys Arg Arg Asn Asp Asn Thr Ile Asp Cys His Tyr Phe
            260                 265                 270

Leu Pro Ile Tyr Cys Asp Asp Met Glu Phe Tyr Asn Glu Met Gln Val
        275                 280                 285

Tyr His Asn Asn Ile Val Lys Pro Glu Met Ser Val Thr Leu Gly Leu
    290                 295                 300

Pro Lys Ser
305
```

<210> 9
<211> 1437
<212> DNA
<213> Yersinia pestis

<400> 9

```
atggcagatt taactgcaag caccactgca acggcaactc ttgttgaacc agcccgcatc      60
actcttacat ataaggaagg cgctccaatt acaattatgg acaatggaaa catcgataca     120
gaattacttg ttggtacgct tactcttggc ggctataaaa caggaaccac tagcacatct     180
gttaacttta cagatgccgc gggtgatccc atgtacttaa catttacttc tcaggatgga     240
aataaccacc aattcactac aaaagtgatt ggcaaggatt ctagagattt tgatatctct     300
```

```
cctaaggtaa acggtgagaa ccttgtgggg gatgacgtcg tcttggctac gggcagccag      360
gatttctttg ttcgctcaat tggttccaaa ggcggtaaac ttgcagcagg taaatacact      420
gatgctgtaa ccgtaaccgt atctaaccaa gaattcatga ttagagccta cgaacaaaac      480
ccacaacatt ttattgagga tctagaaaaa gttagggtgg aacaacttac tggtcatggt      540
tcttcagttt tagaagaatt ggttcagtta gtcaaagata aaaatataga tatttccatt      600
aaatatgatc ccagaaaaga ttcggaggtt tttgccaata gagtaattac tgatgatatc      660
gaattgctca agaaaatcct agcttatttt ctacccgagg atgccattct taaaggcggt      720
cattatgaca accaactgca aaatggcatc aagcgagtaa aagagttcct tgaatcatcg      780
ccgaatacac aatgggaatt gcgggcgttc atggcagtaa tgcatttctc tttaaccgcc      840
gatcgtatcg atgatgatat tttgaaagtg attgttgatt caatgaatca tcatggtgat      900
gcccgtagca agttgcgtga agaattagct gagcttaccg ccgaattaaa gatttattca      960
gttattcaag ccgaaattaa taagcatctg tctagtagtg gcaccataaa tatccatgat     1020
aaatccatta atctcatgga taaaaattta tatggttata cagatgaaga gatttttaaa     1080
gccagcgcag agtacaaaat tctcgagaaa atgcctcaaa ccaccattca ggtggatggg     1140
agcgagaaaa aaatagtctc gataaaggac tttcttggaa gtgagaataa aagaaccggg     1200
gcgttgggta atctgaaaaa ctcatactct tataataaag ataataatga attatctcac     1260
tttgccacca cctgctcgga taagtccagg ccgctcaacg acttggttag ccaaaaaaca     1320
actcagctgt ctgatattac atcacgtttt aattcagcta ttgaagcact gaaccgtttc     1380
attcagaaat atgattcagt gatgcaacgt ctgctagatg acacgtctgg taaatga        1437
```

<210> 10
<211> 478
<212> PRT
<213> Yersinia pestis

<400> 10

```
Met Ala Asp Leu Thr Ala Ser Thr Thr Ala Thr Ala Thr Leu Val Glu
1               5                   10                  15

Pro Ala Arg Ile Thr Leu Thr Tyr Lys Glu Gly Ala Pro Ile Thr Ile
            20                  25                  30

Met Asp Asn Gly Asn Ile Asp Thr Glu Leu Leu Val Gly Thr Leu Thr
        35                  40                  45
```

```
Leu Gly Gly Tyr Lys Thr Gly Thr Thr Ser Thr Ser Val Asn Phe Thr
    50                  55                  60

Asp Ala Ala Gly Asp Pro Met Tyr Leu Thr Phe Thr Ser Gln Asp Gly
65                  70                  75                  80

Asn Asn His Gln Phe Thr Thr Lys Val Ile Gly Lys Asp Ser Arg Asp
                85                  90                  95

Phe Asp Ile Ser Pro Lys Val Asn Gly Glu Asn Leu Val Gly Asp Asp
            100                 105                 110

Val Val Leu Ala Thr Gly Ser Gln Asp Phe Phe Val Arg Ser Ile Gly
        115                 120                 125

Ser Lys Gly Gly Lys Leu Ala Ala Gly Lys Tyr Thr Asp Ala Val Thr
    130                 135                 140

Val Thr Val Ser Asn Gln Glu Phe Met Ile Arg Ala Tyr Glu Gln Asn
145                 150                 155                 160

Pro Gln His Phe Ile Glu Asp Leu Glu Lys Val Arg Val Glu Gln Leu
                165                 170                 175

Thr Gly His Gly Ser Ser Val Leu Glu Glu Leu Val Gln Leu Val Lys
            180                 185                 190

Asp Lys Asn Ile Asp Ile Ser Ile Lys Tyr Asp Pro Arg Lys Asp Ser
        195                 200                 205

Glu Val Phe Ala Asn Arg Val Ile Thr Asp Asp Ile Glu Leu Leu Lys
    210                 215                 220

Lys Ile Leu Ala Tyr Phe Leu Pro Glu Asp Ala Ile Leu Lys Gly Gly
225                 230                 235                 240

His Tyr Asp Asn Gln Leu Gln Asn Gly Ile Lys Arg Val Lys Glu Phe
                245                 250                 255

Leu Glu Ser Ser Pro Asn Thr Gln Trp Glu Leu Arg Ala Phe Met Ala
            260                 265                 270

Val Met His Phe Ser Leu Thr Ala Asp Arg Ile Asp Asp Asp Ile Leu
        275                 280                 285
```

```
Lys Val Ile Val Asp Ser Met Asn His His Gly Asp Ala Arg Ser Lys
    290                 295                 300

Leu Arg Glu Glu Leu Ala Glu Leu Thr Ala Glu Leu Lys Ile Tyr Ser
305                 310                 315                 320

Val Ile Gln Ala Glu Ile Asn Lys His Leu Ser Ser Ser Gly Thr Ile
                325                 330                 335

Asn Ile His Asp Lys Ser Ile Asn Leu Met Asp Lys Asn Leu Tyr Gly
            340                 345                 350

Tyr Thr Asp Glu Glu Ile Phe Lys Ala Ser Ala Glu Tyr Lys Ile Leu
        355                 360                 365

Glu Lys Met Pro Gln Thr Thr Ile Gln Val Asp Gly Ser Glu Lys Lys
    370                 375                 380

Ile Val Ser Ile Lys Asp Phe Leu Gly Ser Glu Asn Lys Arg Thr Gly
385                 390                 395                 400

Ala Leu Gly Asn Leu Lys Asn Ser Tyr Ser Tyr Asn Lys Asp Asn Asn
                405                 410                 415

Glu Leu Ser His Phe Ala Thr Thr Cys Ser Asp Lys Ser Arg Pro Leu
            420                 425                 430

Asn Asp Leu Val Ser Gln Lys Thr Thr Gln Leu Ser Asp Ile Thr Ser
        435                 440                 445

Arg Phe Asn Ser Ala Ile Glu Ala Leu Asn Arg Phe Ile Gln Lys Tyr
    450                 455                 460

Asp Ser Val Met Gln Arg Leu Leu Asp Asp Thr Ser Gly Lys
465                 470                 475
```

**Description**

Cross Reference to related applications

This application is a CIP of U.S. provisional application no. 61/056,365; filed May 27, 2008, to which priority is claimed under 35 USC 119.

Government Rights

Development of the invention was supported in part by the USDA 3611-21000-017-00D and NIH R01 GM 63879 grants. The U.S. government has certain rights in the invention.

BACKGROUND

*Yersinia pestis* has caused three plague pandemics and killed approximately 200 million people (62). At least 2,000 cases of plague are reported annually by the World Health Organization http://www.who.int/mediacentre/factsheets/fs267/en/index.html), including several recent outbreaks in India. The nonavailability of a human plague vaccine is a public health concern, given the potential use of *Y. pestis* as an agent for bioterrorism (42). Since the lungs and respiratory tract are vulnerable to a first exposure to *Y. pestis,* adequate protective immunity-achieved either by transudation of high levels of circulating immunoglobulin G (IgG), by induction of local immunity (IgA), or by a combination of both-must be available. Indeed, protection against both s.c. (95) and aerosolized (3, 93) *Y. pestis* challenges was found to be associated with F1-V-specific IgG1, a TH2-associated antibody. Systemic IgG is a known consequence of parenteral immunization, and many studies have demonstrated the efficacy of s.c. and intramuscular vaccines for providing protection against pathogen challenge (for a review, see reference 94). However, intranasal or even oral delivery of subunit vaccines may be more effective because of the ability of such vaccines to elicit protective immunity directly at the mucosal surface. To date, there is no known example of a plant-derived vaccine against Yersinia pestis.

The establishment of successful protocols for oral vaccination could radically alter the current landscape of infectious diseases. Oral delivery of plant-derived vaccine antigens could eliminate expensive fermentation and purification systems, cold storage and transportation steps, and delivery via sterile needles, significantly reducing costs. Plant-derived oral vaccines have other distinct advantages, including the ability to stimulate both systemic and mucosal immune responses, facilitating large-scale production and simplified storage (eliminating frozen stocks), improving safety due to the lack of human pathogens or microbial toxin contamination, protecting therapeutic proteins by bioencapsulation, and delivering these proteins to the gut-associated lymphoid tissue (7, 15, 80).

BRIEF DESCRIPTION OF THE DRAWINGS

**FIG. 1.** Evaluation of transgene integration and homoplasmy in the To (first) generation of both cultivars. DNA extracted from transformed and untransformed plants were probed with the flanking sequence probe (A). Southern blot analysis of transgenic lines in lanes 1 to 3 (Petit Havana) and lanes 5 to 7 (LAMD) produced restriction fragments that were 9.5 and 1.5 kb long (B). Wild-type Petit Havana (lane 4) and LAMD (lane 8) plants produced only the 8-kb fragment.

**FIG. 2.** Quantitation of chloroplast-synthesized F1-V by ELISA. The quantity of F1-V is expressed as a percentage of the TSP. For continuous illumination, leaf material was sampled on days 0, 1, 3, and 5 for young, mature, and old leaves.

**FIG. 3.** Enrichment of the chloroplast-derived F1-V antigen. Transgenic plant crude extracts were lyophilized and then sequentially centrifuged through 50- and 100-kDa MWCO columns. (A) Immunoblot analysis of lyophilized crude extracts. Lanes 1 to 5, dilutions of lyophilized crude extract (1:1, 1:10, 1:100, 1:1,000, and 1:5,000); lanes 6 and 7, 750 and 250 ng, respectively, of recombinant F1-V purified from *E. coli.* (B) Immunoblot analysis of enriched plant extracts analyzed by SDS-PAGE, followed by immunoblotting. Lanes 1 to 3, dilutions of > 100-kDa column retentate (1:5, 1:10, and 1:50); lanes 4 to 6, dilutions of >100-kDa column wash (1:5, 1:10, and 1:50); lane 7, 750 ng of recombinant F1-V purified from *E. coli.* (C) Chloroplast-derived enF1-V samples were analyzed by SDS-PAGE, followed by staining with Coomassie blue. Lanes 1 to 5, dilutions of lyophilized crude extract (1:5, 1:10, 1:100, 1:500, and 1:50,000); lanes 6 and 7, 750 and 250 ng, respectively, of recombinant F1-V purified from *E. coli*; lanes M, molecular weight markers.

**FIG. 4.** Quantitation of serum antibody titers after immunization. Mice were immunized as described in the text. On days 21, 43, and 140, blood was collected and the serum was analyzed for the presence of (A) F1-specific IgG1, (B) V-specific IgG1, (C) F1-V-specific IgG1, (D) F1-V-specific IgG2a, and (E) F1-V-specific IgA antibodies. IgG titers were calculated by determining the reciprocal of the highest dilution that resulted in a difference between each treatment group and untreated group of 0.25 optical density unit; IgA titers were calculated based on a difference between each treatment group and untreated group of 0.1 optical density unit. SC F1-V, s.c. enF1-V prime dose and s.c. enF1-V boosts; Oral F1-V, s.c. enF1-V prime dose and oral F1-V boosts; Oral WT, s.c. enF1-V prime dose and oral wild-type boosts; SC AlH, s.c. AlH prime dose and s.c. AlH boosts. Ab, antibody.

**FIG. 5.** Mice receiving oral boosts of chloroplast-derived F1-V survived longer than mice receiving s.c. boosts. Animals were challenged with 15 $LD_{50}$ of *Y. pestis* CO92 (whole-body $LD_{50}$, 6.8 x $10^4$ CFU), and their survival was monitored. Differences in survival between untreated animals and immunized animals were statistically significant ($P < 0.05$, as determined by the log rank test). Differences between animals boosted s.c. and animals boosted orally were also significant ($P < 0.05$). SC F1-V, s.c. enF1-V prime dose and s.c. enF1-V boosts; Oral F1-V, s.c. enF1-V prime dose and oral F1-V boosts; Oral WT, s.c. enF1-V prime dose and oral wild-type boosts; SC AlH, s.c. AlH prime dose and s.c. AlH boosts.

**FIG. 6** is shows a schematic diagram showing production of a F1-V chloroplast vector for tranforming lettuce chloroplasts FIG. 6A-B; and expression of F1-V in lettuce FIG. 6C-D. All F1-V transplastomic lines showed homoplasmy which contained 6.3 kb fragment without the 3.13 kb fragment observed in untransformed plant (Fig 6C). The expression of F1-V was confirmed by Western blot and showed a prominent band of ~53 kDa corresponding to the size of F1-V fusion protein (Fig 6D)

## DETAILED DESCRIPTION

Human plague vaccines based on either a live, attenuated strain or a killed, whole-cell preparation (for a review, see reference 5) are no longer commercially available. Because of the severity of the infection and the potential of *Y. pestis* as a bioterrorist agent, the inventors have developed a subunit vaccine produced in transgenic tobacco chloroplasts. This vaccine offers the advantage of employing two defined antigens that are able to elicit high-level protection. Of the various *Y. pestis* antigens that have been tested preclinically, the fraction 1 (F1) outer capsular and low-calcium response V (LcrV or V) proteins appear to be the most promising vaccine candidates (9, 31, 64). The F1 protein has been reported to have antiphagocytic capability (21, 68, 89), while LcrV, a major component of the type III secretion system, is required for the production and translocation of *Yersinia* outer proteins, several of which have antihost activities in the eukaryotic host cell (63, 77, 79).

Given the high costs associated with needle-based vaccination, the inventors investigated whether needle-free (i.e., oral) vaccine delivery could provide animals with similar levels of protection against pathogen challenge. It was hypothesized that a heterologous prime-boost strategy for plague may provide improved protection compared with parenteral immunization. We investigated the efficacy of a plague vaccine protocol that incorporates both elements of successful vaccination against *Y. pestis*: s.c. delivery of enriched antigen preparations of F1-V prepared from transgenic, low-nicotine tobacco (enF1-V), followed by oral boosts of antigen expressed in transgenic plants. The efficacy of the vaccine was assessed by aerosol challenge with *Y. pestis.*

However, the intent of this study was not to compare oral boosters with s.c. boosters (by dosage or number of boosters). Experimentally, any such comparison is not possible because oral delivery involves antigens encapsulated in plant cells without any adjuvant, whereas in s.c. delivery antigens bound to an adjuvant are directly delivered to the circulatory system. It is not possible to deliver antigens via oral boosters in a quantitative manner because the release of antigens from plant cells depends on several factors, including the population of bacteria that can degrade the plant cell wall and presentation of an antigen to the gut-associated lymphoid tissue. It is not possible to control these factors in experimental animals in a quantitative manner. An equal number of boosters does not guarantee an equal quantity of antigen delivered. Therefore, this study simply demonstrated that oral boosting via plant cells is a novel mode of delivery of vaccine antigens and might provide a low-cost delivery option, especially for delivery of biodefense vaccines at times of crisis to a very large population or in developing countries where cold storage and transportation of vaccines are major challenges.

The present description discloses vaccines for conferring immunity in mammals to *Y. pestis,* as well as vectors and methods for plastid transformation of plants to produce protective antigens and vaccines for oral delivery. The present description teaches novel expression of therapeutic proteins in chloroplasts, and in particular, chloroplasts of edible plants. Some plants such as potato contain components that make them not suitable for eating raw, but which are edible upon further processing, such as by cooking. In more specific embodiments, the present invention pertains to plant cells from plants edible without cooking, wherein the plant cells comprise chloroplasts transformed to express therapeutic proteins as defined in the claims. In a one embodiment, a F1-LcrV fusion protein antigen is expressed in the chloroplasts of *Lactuca sativa* representing the first demonstration of a therapeutic protein being expressed in chloroplasts of an edible plant. The specification also describes a method of retarding the development or treating of diabetes in a subject in need thereof. The method involves administering to the subject a composition comprising a F1 and/or LcrV polypeptide and a plant remnant from a plant edible without cooking.

The term "a plant edible without cooking" refers to a plant that is edible, i.e., edible without the need to be subjected to heat exceeding 120 deg F for more than 5 min. Examples of such plants include, but are not limited to, Lactuca sativa (lettuce), apple, berries such as strawberries and raspberries, citrus fruits, tomato, banana, carrot, celery, cauliflower; broccoli, collard greens, cucumber, muskmelon, watermelon, pepper, pear, grape, peach, radish and kale. In a specific embodiment, the edible plant is Lactuca sativa.

Edible plants that require cooking or some other processing are not excluded from the teachings herein.

A plant remnant may include one or more molecules (such as, but not limited to, proteins and fragrments thereof, minerals, nucleotides and fragments thereof, plant structural components, etc.) derived from the plant in which the protein of interest was expressed. Accordingly, a composition pertaining to whole plant material (e.g., whole or portions of plant leafs, stems, fruit, etc.) or crude plant extract would certainly contain a high concentration of plant remnants, as well as a composition comprising purified protein of interest that has one or more detectable plant remnants. In a specific embodiment, the plant remnant is rubisco.

In another embodiment, the invention pertains to an administratable composition for eliciting a protective immune response against *Y. pestis.* The composition is defined in the claims.

The specification provides a stable plastid transformation and expression vector which comprises an expression cassette comprising, as operably linked components in the 5' to the 3' direction of translation, a promoter operative in said plastid, a selectable marker sequence, a heterologous polynucleotide sequence coding for a polypeptide comprising at least 70%, 75%, 80%, 85%, 90%, 95%, or 97% identity to a F1 and/or a LcrV protein, transcription termination functional in said plastid, and flanking each side of the expression cassette, flanking DNA sequences which are homologous to a DNA sequence of the target plastid genome, whereby stable integration of the heterologous coding sequence into the plastid genome of the target plant is facilitated through homologous recombination of the flanking sequence with the homologous sequences in the target plastid genome.

Methods, vectors, and compositions for transforming plants and plant cells are taught for example in WO 01/72959; WO 03/057834; and WO 04/005467. WO 01/64023 discusses use of marker free gene constructs.

Proteins expressed in accord with certain embodiments taught herein may be used in vivo by administration to a subject, human or animal in a variety of ways. The pharmaceutical compositions may be administered orally or parenterally, i.e., subcutaneously, intramuscularly or intravenously, though oral administration is preferred.

Oral compositions produced by embodiments of the present invention can be administrated by the consumption of the foodstuff that has been manufactured with the transgenic plant producing the plastid derived therapeutic protein. The edible part of the plant, or portion thereof, is used as a dietary component. The therapeutic compositions can be formulated in a classical manner using solid or liquid vehicles, diluents and additives appropriate to the desired mode of administration. Orally, the composition can be administered in the form of tablets, capsules, granules, powders and the like with at least one vehicle, e.g., starch, calcium carbonate, sucrose, lactose, gelatin, etc. The preparation may also be emulsified. The active immunogenic ingredient is often mixed with excipients which are pharmaceutically acceptable and compatible with the active ingredient. Suitable excipients are, e.g., water, saline, dextrose, glycerol, ethanol or the like and combination thereof. In addition, if desired, the compositions may contain minor amounts of auxiliary substances such as wetting or emulsifying agents, pH buffering agents, or adjuvants. In a preferred embodiment the edible plant, juice, grain, leaves, tubers, stems, seeds, roots or other plant parts of the pharmaceuticalproducing transgenic plant is ingested by a human or an animal thus providing a very inexpensive means of treatment of or immunization against disease.

In a specific embodiment, plant material (e.g. lettuce material) comprising chloroplasts capable of expressing F1 and/or LcrV is homogenized and encapsulated. In one specific embodiment, an extract of the lettuce material is encapsulated. In an alternative embodiment, the lettuce material is powderized before encapsulation.

In alternative embodiments, the compositions may be provided with the juice of the transgenic plants for the convenience of administration. For said purpose, the plants to be transformed are preferably selected from the edible plants consisting of tomato, carrot and apple, among others, which are consumed usually in the form of juice.

According to another embodiment, the subject invention pertains to a transformed chloroplast genome that has been transformed with a vector comprising a heterologous gene that expresses a peptide as disclosed herein.

Of particular present interest is a transformed chloroplast genome that has been transformed with a vector comprising a heterologous gene that expresses a F1 and/or LcrV polypeptide. In a related embodiment, the present specification describes a plant comprising at least one cell transformed to express a peptide as disclosed herein. One specific embodiment relates to expression of a F1-V fusion protein such as that disclosed in US. Patent Pub. 20090130103, and biological variants thereof.

Variants which are biologically active, refer to those, in the case of oral tolerance, that activate T-cells and/or induce a Th2 cell response, characterized by the upregulation of immunosuppressive cytokines (such as IL10 and IL4) and serum antibodies (such as IgG1), or, in the case of desiring the native function of the protein, is a variant which maintains the native function of the protein. Preferably, naturally or non-naturally occurring polypeptide variants have amino acid sequences which are at least about 55, 60, 65, or 70, preferably about 75, 80, 85, 90, 96, 96, or 98% identical to the full-length amino acid sequence or a fragment thereof. Percent identity between a putative polypeptide variant and a full length amino acid sequence is determined using the Blast2 alignment program (Blosum62, Expect 10, standard genetic codes).

In specific embodiments, a variant of a polypeptide is one having at least about 80% amino acid sequence identity with the amino acid sequence of a either a F1 or LcrV antigen. Such variant polypeptides include, for instance, polypeptides wherein one or more amino acid residues are added, or deleted, at the N- and/or C-terminus, as well as within one or more internal domains, of the full-length amino acid sequence. Fragments of the peptides are also contemplated. Ordinarily, a variant polypeptide will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with a polypeptide encoded by a nucleic acid molecule shown in Attachment B or a specified fragment thereof. Ordinarily, variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, or more.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of polypeptide can be found using computer programs well known in the art, such as DNASTAR software. Whether an amino acid change results in a biologically active F1 and/or LcrV polypeptide can readily be determined by assaying for elicitation of immune responses, either determined *in vitro* or *in vivo,* as described for example, in the specific Examples, below.

Reference to genetic sequences herein refers to single- or double-stranded nucleic acid sequences and comprises a coding sequence or the complement of a coding sequence for polypeptide of interest. Degenerate nucleic acid sequences encoding polypeptides, as well as homologous nucleotide sequences which are at least about 50, 55, 60, 65, 60, preferably about 75, 90, 96, or 98% identical to the cDNA may be used in accordance with the teachings herein polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologs, and variants of nucleic acid sequences which encode biologically active polypeptides also are useful polynucleotides.

Variants and homologs of the nucleic acid sequences described above also are useful nucleic acid sequences. Typically, homologous polynucleotide sequences can be identified by hybridization of candidate polynucleotides to known polynucleotides under stringent conditions, as is known in the art. For example, using the following wash conditions: 2 X SSC (0.3 M NaCl, 0.03 M sodium citrate, pH 7.0), 0.1% SDS, room temperature twice, 30 minutes each; then 2X SSC, 0.1% SDS, 50° C. once, 30 minutes; then 2X SSC, room temperature twice, 10 minutes each homologous sequences can be identified which contain at most about 25-30% basepair mismatches. More preferably, homologous nucleic acid strands contain 15-25% basepair mismatches, even more preferably 5-15% basepair mismatches.

Species homologs of polynucleotides referred to herein also can be identified by making suitable probes or primers and screening cDNA expression libraries. It is well known that the Tm of a double-stranded DNA decreases by 1-1.5° C with every 1% decrease in homology (Bonner et al., J. Mol. Biol. 81, 123 (1973). Nucleotide sequences which hybridize to polynucleotides of interest, or their complements following stringent hybridization and/or wash conditions also are also useful polynucleotides. Stringent wash conditions are well known and understood in the art and are disclosed, for example, in Sambrook et al., MOLECULAR CLONING: A LABORATORY MANUAL, 2nd ed., 1989, at pages 9.50-9.51.

Typically, for stringent hybridization conditions a combination of temperature and salt concentration should be chosen that is approximately 12-20° C below the calculated $T_m$ of the hybrid under study. The $T_m$ of a hybrid between a polynucleotide of interest or the complement thereof and a polynucleotide sequence which is at least about 50, preferably about 75, 90, 96, or 98% identical to one of those nucleotide sequences can be calculated, for example, using the equation of Bolton and McCarthy, Proc. Natl. Acad. Sci. U.S.A. 48, 1390 (1962):

$$T_m=81.5^{O}\ C-16.6(\log_{10}\ [Na^+\ ])+0.41(\%\ G+C)-0.63(\%\ formamide)-600/l),$$

where l=the length of the hybrid in basepairs.

Stringent wash conditions include, for example, 4 X SSC at 65° C, or 50% formamide, 4 X SSC at 42° C, or 0.5 X SSC, 0.1% SDS at 65° C. Highly stringent wash conditions include, for example, 0.2 X SSC at 65° C.

According to another embodiment, the invention pertains to a method of producing a Y. pestis antigen containing composition, the method including: obtaining a stably transformed Lactuca sativa plant which includes a plastid stably transformed with an expression vector which has an expression cassette having, as operably linked components in the 5' to the 3' direction of translation, a promoter operative in a plastid operably linked to a Lactuca sativa psbA 5'UTR, a selectable marker sequence, a heterologous polynucleotide sequence coding for a F1 antigen, a LcrV antigen or a F1-LcrV fusion protein or a sequence having at least 90%) identity to said F1 antigen, a LcrV antigen or F1-LcrV fusion protein encoding sequence, a transcription termination sequence functional in said plastid, said expression cassette being flanked by sequences from trnI and trnA genes for homologous recombination of said expression cassette into the intergenic region between said trnI and trnA genes of said Lactuca sativa chloroplast genome; and homogenizing material of said stably transformed Lactuca sativa plant to produce homogenized material.

EXAMPLES

**Example 1: Methods and Materials related to Examples 2-6**

**Construction of pLDS-F1V and regeneration of transgenic lines.** The F1-V fusion gene in a pET-24 vector (pPW731, obtained from the United States Army Medical Research Institute of Infectious Diseases [USAMRIID]) was isolated by cleavage with the restriction enzymes NdeI and NotI. The fragment was subcloned into a pCR vector containing the 5' untranslated region (UTR) of the *psbA* gene and then inserted (using NotI and EcoRV) into the universal chloroplast vector pLD to create pLDS-F1V. The chloroplast expression vector pLDS-F1V was bombarded into Petit Havana and LAMD (low-nicotine variety) *Nicotiana tabacum* leaves as described elsewhere (48, 85, 86). Transgenic shoots were first tested by PCR to confirm transgene integration. Plants that were confirmed to contain the F1-V transgene were transferred to pots and were grown with a photoperiod consisting of 16 of h light and 8 h of darkness in a growth chamber at 26°C or in a greenhouse.

**Southern blot analysis.** Total plant DNA was extracted from tobacco plants using a DNeasy plant mini kit (Qiagen, Valencia, CA). Total plant DNA was digested with BamHI and hybridized with the flanking sequence probe, which was obtained from the pUC-Ct vector by digesting it with BamHI and BglII, which yielded a 0.81-kb fragment. The probe was prepared by random primed $^{32}$P labeling (Ready-To-Go DNA labeling beads; Amersham Biosciences, Pittsburgh, PA). The probe was hybridized to the membrane using the Quick-hyb solution and protocol (Stratagene, La Jolla, CA). The radiolabeled blots were exposed to X-ray film and then developed.

**Immunoblot analysis.** The immunoblot analysis protocol has been described previously (48, 85, 86). Plant extraction buffer (PEB) (48, 85, 86) was made fresh on the day of the analysis. Extraction was performed using a ratio of 100 mg of leaf material to 200 $\mu$l of PEB. Transgenic protein was detected using polyclonal serum raised against F1 in rabbits (USAMRIID).

**ELISA.** Dilutions of plant crude extracts ranging from 1:5 to 1:5,000 were made in coating buffer (48, 85, 86). Recombinant F1-V (standard) was also diluted in coating buffer. An indirect ELISA was performed as described previously (48, 85, 86). The transgenic protein was detected using the anti-Fl polyclonal primary antibody.

**Estimation of TSP.** The total soluble protein (TSP) in plant crude extracts was determined by the Bio-Rad protein assay as previously described (48, 85, 86). Bovine serum albumin (Sigma Chemical, St. Louis, MO) was used as a standard at concentrations ranging from 0.05 to 0.5 mg/ml.

**Lyophilization and enrichment of transgenic crude extracts.** To concentrate the soluble protein in transgenic leaf material, 28.99 g of transgenic leaf material was extracted in 75 ml of PEB. Aliquots (10 ml) were transferred to 50-ml conical tubes and lyophilized to obtain a final volume of 1.5 ml. The concentrated extracts were then pooled, loaded onto Centricon 50-kDa molecular weight cutoff (MWCO) columns (Millipore, Billerica, MA), and centrifuged for 10 min at 5,000 x g. The flowthrough fraction was collected and run through the same column a second time. The retentate fractions were collected, pooled, and loaded onto 100-kDa MWCO columns, and the process was repeated. All flowthrough and retentate fractions were analyzed for the presence of F1-V by sodium dodecyl sulfate-polyacrylamide gel electrophoresis (SDS-PAGE), immunoblot analysis, and ELISA.

**Adsorption of enF1-V protein to adjuvant.** F1-V enriched from transgenic tobacco crude extract (enF1-V) was mixed with Alhydrogel (AlH) (Sigma Chemical, St. Louis, MO) diluted 1:4 in phosphate-buffered saline (PBS) and incubated at 4°C with gentle rocking overnight. The samples were then centrifuged at 2,000 x g for 5 min at 4°C, and the protein-adsorbed pellet was resuspended in PBS to a final concentration of 250 μg/ml. The adsorption efficiency was calculated on the basis of the total amount of protein added to the adjuvant compared with the protein remaining in the supernatant after adsorption.

**Immunization.** Female Hsd:ND4 Swiss Webster mice weighing 18 to 20 g each were purchased from Harlan Sprague Dawley (Indianapolis, IN). Mice were divided into the following five treatment groups: group 1, mice given s.c. enF1-V prime and s.c. enF1-V boost doses (s.c. F1-V group) (10 animals); group 2, mice given s.c. enF1-V prime and oral F1-V boost doses (oral F1-V group) (10 animals); group 3, mice given s.c. enF1-V prime and oral wild-type boost doses (oral WT group) (10 animals); group 4, mice given s.c. AlH prime and s.c. AlH boost doses (s.c. AlH group) (5 animals); and group 5, untreated mice (5 animals).

s.c. injections were delivered as follows. Doses of chloroplast-derived enF1-V adsorbed to AlH were diluted in 200 μl of PBS and injected into the scruff of the neck using a tuberculin syringe equipped with a 27-gauge needle. A single 25-μg dose of enF1-V was delivered on day 0 as the vaccine prime dose to animals in groups 1 to 3; boosts of 10 μg of enF1-V per dose were given to animals in group 1. Group 4 animals received equivalent amounts of AlH in the absence of F1-V. Mice in groups 1 and 4 received four s.c. boosts, on days 14, 28, 126, and 164.

The doses used for oral delivery were prepared as follows. Leaf material from transgenic or wild-type plants was ground in liquid nitrogen in cold, autoclaved mortars and pestles. The pulverized leaf material was stored at -80°C until the day of immunization. Oral doses (500 mg each) of either transgenic (group 2) or nontransgenic (group 3) leaf material were resuspended in sterile PBS (250 μl) and homogenized for 5 min with an OMNI International GLH-2596 probe to disperse the plant cells. The plant cell suspension (without clumps) was stored on ice until oral gavage. The oral doses were delivered by using a tuberculin syringe equipped with a 20-gauge bulb-tipped gastric gavage needle. Mice in groups 2 and 3 received eight oral boosts, on days 8, 15, 22, 29, 119, 126, 164, and 171. Mice were shipped to USAMRIID on day 182.

**Determination of antibody titers.** Blood samples were obtained on day 7 before vaccination and days 21, 43, and 140 after vaccination. Blood was collected from the retroorbital plexus of anesthetized mice (4% isoflurane) in Microtainer serum separation tubes (Becton-Dickinson, Franklin Lakes, NJ). The samples were allowed to clot for a minimum of 30 min at room temperature and then centrifuged at 13,000 x *g* for 2 min. The serum was transferred to fresh tubes and either placed on ice or stored at -80°C.

Serum levels of F1-, V-, and F1-V-specific IgG1 and F1-V-specific IgG2a and IgA were determined by ELISA. Purified recombinant F1, V, or F1-V (100 ng), diluted in coating buffer, was incubated overnight at 4°C. Fivefold dilutions of serum (beginning with 1:100 in PBS) were then aliquoted and incubated overnight at 4°C. The secondary antibody was either anti-mouse IgG1 or IgG2a or IgA. To compare the levels of antibodies of different isotypes, dilutions of purified IgG1, IgG2a, and IgA were incubated overnight at 4°C, followed by addition of secondary antibody (anti-IgG1, anti-IgG2a, and anti-IgA, respectively).

**Bacterial challenge.** *Y. pestis* CO92 was prepared and used in accordance with a previously reported procedure (2). Briefly, bacteria were harvested from tryptose blood agar base (Difco Laboratories, Detroit, MI) slants and inoculated into 5 ml of heart infusion broth (HIB) (Difco), and the concentration was adjusted to an optical density at 620 nm of 1.0 (approximately $10^9$ CFU/ml). For aerosol challenges, 2 ml of the HIB bacterial suspension was used to inoculate flasks containing 100 ml HIB supplemented with 0.2% xylose. The broth cultures were grown for 24 h in a 28°C shaker at 100 to 150 rpm. The concentration of pelleted cells was adjusted to an optical density at 620 nm of 10.0 (approximately $10^{10}$ CFU/ml), and the preparations were diluted to produce the aerosolized doses reported below. Antifoam agent A (Sigma) was added to a final concentration of 0.2% (vol/vol) to the bacterial suspension just before the aerosol challenges. The aerosol challenges were conducted by whole-body exposure of mice to a small-particle aerosol (median diameter, 1.2 μm). Up to 40 unanesthetized mice were challenged simultaneously inside a class III biological safety cabinet. Mice from various groups were divided into different cages to minimize exposure differences. The inhaled doses for each exposure were estimated using Guyton's formula (33). Mice were observed twice daily for 21 days after exposure for signs of morbidity or mortality. Any mouse found to be recumbent was humanely euthanized.

**Calculation of bacterial burden.** Homogenates (10%) of whole spleen tissue were plated in duplicate at the indicated dilutions on sheep blood agar plates. The plates were incubated at 28°C for 48 h, and colonies were counted. The total bacterial burden per gram of spleen was calculated by multiplying the plate count by the appropriate dilution factor.

**Statistical analysis.** Pearson analysis was conducted to calculate correlation coefficients comparing survival postchallenge and individual mouse day 140 postprime immunization F1-V-specific antibody titers. Fisher exact tests with step-down Bonferroni adjustments were used to compute statistical differences between observed survival rates.

Survival curves were processed using Kaplan-Meier survival analysis with log rank tests and step-down Bonferroni adjustments. Statistical significance was considered a *P* value of <0.05, as indicated below.

**Care and treatment of animals.** This research was conducted in compliance with the Animal Welfare Act and other federal statutes and regulations relating to animals and experiments involving animals and adhered to principles stated in the *Guide for the Care and Use of Laboratory Animals* (National Research Council, 1996). The animals received food and water ad libitum for the duration of the study.

**Example 2:Assessment of transgenic plants.** The F1-V fusion gene was cloned into the universal chloroplast vector pLD-CtV to produce the final vector, pLDS-F1V (Fig. 1A), which was used for particle bombardment. In this construct, the *trnI* and *trnA* genes were used as flanking sequences for homologous recombination with the native chloroplast genome. The *aadA* gene conferring spectinomycin resistance was used for selection. True chloroplast transformants were distinguished from nuclear transformants and mutants by PCR (data not shown). PCR was also employed to ensure integration of the F1-V fusion gene (data not shown). Confirmed Petit Havana and LAMD (12) transgenic shoots were transferred to rooting medium and then to either growth chambers or the greenhouse. In order to evaluate homoplasmy, total DNA extracted from untransformed and transgenic lines was probed with chloroplast flanking sequences (Fig. 1A). Homoplasmic lines of both cultivars contained both 9.5- and 1.5-kb fragments without the 8-kb fragment observed in untransformed lines (Fig. 1B).

The light-regulated 5' UTR and promoter of the *psbA* gene were used to enhance transcription and translation; the 3' UTR conferred transcript stability. The prokaryotic chloroplast favors AT-rich sequences, which reflects the tRNA abundance. Therefore, the F1-V fusion gene, containing 65% AT, was expected to be expressed well in chloroplasts. Mature leaves always had the highest level of expression of F1-V, and the highest content was on day 3 with continuous illumination (an average of 14.8% of the TSP) (Fig. 2). The *psbA* 5' UTR accounted for both the high expression of F1-V and the change in expression during continuous illumination.

**Example 3:Enrichment of F1-V.** Pooled chloroplast transgenic lines containing a mixture of young, mature, and old leaves were further analyzed for protein expression. Crude extracts of 223 mg of transgenic leaf material contained about 11 mg/ml of TSP or 404 μg/ml of F1-V, equivalent to 3.68% of the TSP or 1.01 mg F1-V per g of freeze-dried leaf material. The lyophilization procedure did not degrade the F1-V protein; no cleaved product was detected in lyophilized crude extracts by immunoblot analysis (Fig. 3A). The lyophilized extract was then enriched for F1-V using MWCO columns. Immunoblot analysis of the various fractions indicated that F1-V was present in the >100-kDa fraction but not in the 50-to 100-kDa fraction (Fig. 3B). This may have been due to protein aggregation, which is commonly observed in transgenic leaf extracts due to high concentrations. Alternatively, it has been reported that during purification F1-V antigens exist principally as dimers and tetramers when they are expressed in *E. coli* (66). Dilutions of enF1-V were analyzed by SDS-PAGE, and there was a prominent band at ~53 kDa (Fig. 3C), corresponding to the size of the F1-V fusion protein. A comparison of the F1-V content of leaf crude extracts with chloroplast-derived enF1-V demonstrated that the enrichment procedure resulted in a nearly 80% improvement in the concentration of transgenic protein in the sample (3.68% versus 18.23% of he TSP).

**Example 4: Animal vaccinations.** Mice were distributed across five treatment groups, four of which received primary s.c. vaccinations. s.c. doses contained enF1-V adsorbed to AlH as an adjuvant, which has been shown to increase the availability and stability of an antigen(s) (32). Each of the treated animals received an s.c. priming dose, which served to induce an initial immune response in groups 1 to 3 to F1-V. Groups of mice received either four s.c. boosts or eight oral boosts. Oral doses (oral F1-V group) were delivered twice as frequently as s.c. doses because of the reduced efficiency of antigen processing by the gut mucosa (72). In addition, as s.c. boosts were delivered with an adjuvant, doubling the number of oral doses was considered adequate compensation for the absence of mucosal adjuvant.

We determined the serum titers of antigen-specific IgG1, IgG2a, and IgA antibodies at various time points. Although mice were immunized with the F1-V fusion protein, we assessed the IgG1 levels based on specificity to three discrete antigens, F1, V, and F1-V. No animal had detectable levels of F1-, V-, or F1-V-specific IgG1 in its preimmune serum (Fig. 4A, 4B, and 4C, respectively), indicating that there was no prior exposure to *Y. pestis.* There was no statistically significant difference between the F1- and V-specific IgG1 titers at any of the time points tested ($P > 0.05$ in all cases).

Following the primary immunization mice received a series of either four s.c. or eight oral boosts. Throughout the schedule, there was an increase in the IgG titers in vaccinated mice, which peaked at day 140 (Fig. 4). The only exception was F1-specific IgG1, whose titers were similar at all time points tested (Fig. 4A). An analysis of the binding affinities of the secondary antibodies indicated that the anti-IgG1 antibody bound purified IgG1 with approximately 40% greater affinity than it bound the IgG2a antibody pair (data not shown). Serum antibody titers were therefore adjusted to reflect the different binding affinities.

Mice in the s.c. F1-V and oral F1-V groups had significantly higher (~2 logs) serum F1-V-specific IgG1 titers than IgG2a titers (compare Fig. 4C and 4D). The development of high titers of circulating IgG1 antibody was associated with protection ($r = 0.71$), while the development of circulating IgG2a antibody was not ($r = 0.15$). Of the three mice in the s.c. F1-V group with the highest day 140 IgG1 titers, two survived (Fig. 4C and Table 1). Fecal IgA levels were also assessed and were below the limit of detection for most animals (data not shown). However, we did observe serum levels of F1-V-specific IgA (Fig. 4E). The IgA titers did not vary significantly ($P > 0.05$) between groups and were weakly correlated with protection ($r = 0.26$).

**Example 5:Aerosol challenge.** The 50% lethal dose ($LD_{50}$) for a Swiss Webster mouse for whole-body exposure to aerosolized *Y. pestis* CO92 is 6.8 x $10^4$ inhaled CFU. The calculated inhaled dose for each mouse was 1.02 x $10^6$ CFU or 15 $LD_{50}$. Doses in this range have been used previously for successful aerosol *Y. pestis* challenge (3, 36, 75).

Mice were challenged on day 189. Following aerosol challenge, mice were observed twice daily for *Y. pestis*-dependent morbidity and mortality. The challenge dose was sufficient to induce death in untreated control animals, and the mean time to death (MTD) was 3 days (Fig. 5), which is consistent with our previous observations for untreated mice (35). Gross pathological examination of mice that succumbed to the challenge revealed significant lung damage consistent with primary plague pneumonia (data not shown). Lungs of mice that survived until the end of the observation period appeared to be grossly normal (data not shown).

All control animals died as a result of pathogenic *Y. pestis* challenge by day 8 postchallenge. At that time, three of nine mice (33%) that received enF1-V delivered as s.c. boosts with adjuvant survived (Fig. 5). The protection was statistically significant compared to mice that received adjuvant only ($P = 0.0438$) or untreated controls ($P = 0.0411$). Seven of eight mice (88%) in the oral F1-V group survived until the end of the challenge experiment (day 22), even without adjuvant. The single mouse that succumbed during the observation period died on day 14 (while the MTD for control animals was 3 days), and this animal had a bacterial count of 1.60 x $10^7$ CFU/g, which was several logs less than the count for control animals (mean bacterial count, 2.17 x $10^{10}$ CFU/g). Both survival and MTD were statistically significant when the mice were compared to all three control groups ($P < 0.0001$). There was also a statistically significant difference between the oral F1-V and s.c. F1-V groups in terms of survival rate and MTD ($P < 0.0001$). Mice that survived the observation period were found to be free of infection by direct plating of spleen tissue (Table 1). The plant-derived vaccine appeared to reduce the bacterial burden in vaccinates that did succumb to infection. A comparison of the *Y. pestis* CFU counts for the spleens of control animals and vaccinates showed that there was an approximately 2-log reduction in the mean bacterial burden of the vaccinates (Table 1).

**Discussion related to Examples 1-5**

The study demonstrated that oral booster doses of *Y. pestis*-derived antigens were at least as effective at eliciting protective antigen-specific antibody responses as needle-based s.c. doses of the same antigens. In addition, it was found for the first time that a plant-based vaccine against the etiologic agent of plague successfully protected mice from lethal *Y. pestis* challenge. The levels of F1-V in chloroplasts-up to 14.8% of the TSP-enabled the delivery of sufficient amounts of vaccine antigens in intact plant cells.

It was observed that oral boosts of transgenic plant material containing the plague fusion antigen F1-V without adjuvant performed as well as s.c. boosts containing chloroplast-derived enF1-V with adjuvant at eliciting a predominant IgG1 titer in the serum of vaccinates. This type of response is indicative of an ongoing TH2 response (78) and, in s.c. vaccinated animals, is typical of AlH-adjuvanted vaccines (32, 53). The TH2 response, specifically mediated by serum levels of F1-V-specific IgG1, has been shown to protect against both s.c. (95) and aerosolized (3, 93) *Y. pestis* challenge. The results of the studycorroborate these findings. Orally boosted mice had similar or, in some cases, higher levels of antigen-specific IgG1. Further, these responses were consistent across the various antigens tested, F1, V, and F1-V. The anti-Fl and anti-V IgG1 responses were not significantly different from one another ($P > 0.05$), indicating that the individual components of the F1-V fusion protein had relatively equal immunogenicities.

The IgG1 levels were generally 2 to 3 logs higher than the corresponding IgG2a levels. Not surprisingly, animals with the highest IgG1 titers were more likely to survive challenge with live *Y. pestis* ($r = 0.71$) (Table 1). Overall, oral F1-V boosters yielded somewhat higher IgG1 titers, more survivors, and a longer MTD than s.c. F1-V boosters. Oral WT group control-boosted mice had the lowest IgG1 titers of the three vaccinated mice, the fewest survivors, and the shortest MTD. Collectively, these results may suggest that the route of immunization or the adjuvant plays a critical role in driving the formation of a protective response in which both IgG1 and IgG2a are present. It is important to note, however, that the oral boosts in our study did not contain adjuvant, nor was the acidic pH of the gut neutralized, which has been done in other oral vaccination schemes (50, 96).

Because of the severe pathogenicity of *Y. pestis,* treatment of plague is a high priority. The use of antimicrobial agents began in 1938 (65) and has led to a dramatic drop in human mortality. Today, the worldwide fatality rate attributable to plague has fallen to less than 8% (26). Natural isolates of *Y. pestis* are uniformly susceptible to all antimicrobial agents active against gram-negative bacteria (8, 26). However, a "natural" strain resistant to multiple antibiotics was isolated in 1995 in the Ambalavao district of Madagascar (27). The possibility of the occurrence of such multidrug-resistant strains in the natural environment, the ease of generating such strains under laboratory conditions (39, 41), and the potential use of such strains for bioterrorist attack, together with the rapidity and high lethality of the disease (for a review, see reference 5), indicate that it is necessary to search for alternatives to antibiotics.

Immunization is now one of the major approaches being pursued to deal with potential *Y. pestis* infection. Use of the serum of vaccinated rabbits to cure animals infected with *Y. pestis* was first attempted more than 100 years ago (97). Since then, several antigens have been shown to be able to produce protective immunity. Among these antigens are the F1 capsular (58, 76) and LcrV (or V) antigens (51, 61, 84), both of which also contain immunodominant epitopes (38, 73, 74, 98). Passive administration of antibodies against target antigens protects macrophages from *Y. pestis*-induced cell death, promotes phagocytosis (13, 64, 88), and protects animals against both bubonic plague and pneumonic plague (4, 25, 37, 61, 69, 84, 91). However, therapy based on a single antibody against a single antigen or epitope will be ineffective in the case of infection with a virulent strain lacking the antigen or expressing a different serological variant of the antigen (6, 25, 69).

**Example 6: Expression of F1-V in lettuce chloroplasts.**

Plague vaccine antigens F1-V have been successfully expressed in lettuce chloroplasts. The pUC based *Lactuca sativa* long flanking plasmid sequence was used to integrate foreign genes into the intergenic spacer region between the *trn*I (Ile) and *trn*A (Ala) genes. In this construct, 16S/*trn*I and *trn*A/23S genes were used as flanking sequences for homologous recombination with the native chloroplast genome (Fig 6A). The lettuce native 16s ribosomal operon promoter, and 3' *rbc*L were amplified from the lettuce chloroplast genome and used to regulate the expression of *aad*A. The *aad*A expression cassette was integrated into the long flanking plasmid and resulted into pLsDV vector. The F1-V sequence was amplified using pLDS-F1V (Arlen et al., 2008) vector as the template. The final F1-V expression cassette with the lettuce *psbA* promoter including 5' untranslated regions (UTR) and the lettuce *psbA* 3' UTR was cloned into pLsDV vector resulting in the lettuce chloroplast vector pLsDV LsF1V (Fig 6B). Lettuce leaf explants were bombarded with pLsDV LsF1V vector. Five to six spectinomycin resistant shoots were observed from ten bombardments after three weeks of selection. PCR analysis confirmed the site specific integration of transgene cassette into the lettuce chloroplast genome. Further, Southern blot analysis was adopted to evaluate site-specific transgene integration and homoplasmy. Total DNA extracted from untransformed and transplastomic lettuce plants was digested with *Sma*I and hybridized with a 1.13 kb *trn*I-*trn*A $^{32}$P-labeled flanking probe prepared from pLsDVF2 vector containing only trnI and trnA sequence of lettuce native plastome. All F1-V transplastomic lines showed homoplasmy which contained 6.3 kb fragment without the 3.13 kb fragment observed in untransformed plant (Fig 6C). The expression of F1-V was confirmed by Western blot and showed a prominent band of ~53 kDa corresponding to the size of F1-V fusion protein (Fig 6D).

References

1. Alvarez, M. L., H. L. Pinyerd, J. D. Crisantes, M. M. Rigano, J. Pinkhasov, A. M. Walmsley, H. S. Mason, and G. A. Cardineau. 2006. Plant-made subunit vaccine against pneumonic and bubonic plague is orally immunogenic in mice. Vaccine 24:2477-2490.

2. Anderson, G. W., Jr., D. G. Heath, C. R. Bolt, S. L. Welkos, and A. M. Friedlander. 1998. Short- and long-term efficacy of single-dose subunit vaccines against Yersinia pestis in mice. Am. J. Trop. Med. Hyg. 58:793-799.

3. Anderson, G. W., Jr., S. E. Leary, E. D. Williamson, R. W. Titball, S. L. Welkos, P. L. Worsham, and A. M. Friedlander. 1996. Recombinant V antigen protects mice against pneumonic and bubonic plague caused by F1-capsule-positive and -negative strains of Yersinia pestis. Infect. Immun. 64:4580-4585.

4. Anderson, G. W., Jr., P. L. Worsham, C. R. Bolt, G. P. Andrews, S. L. Welkos, A. M. Friedlander, and J. P. Burans. 1997. Protection of mice from fatal bubonic and pneumonic plague by passive immunization with monoclonal antibodies against the F1 protein of Yersinia pestis. Am. J. Trop. Med. Hyg. 56:471-473.

5. Anisimov, A. P., and K. K. Amoako. 2006. Treatment of plague: promising alternatives to antibiotics. J. Med. Microbiol. 55:1461-1475.

6. Anisimov, A. P., L. E. Lindler, and G. B. Pier. 2004. Intraspecific diversity of Yersinia pestis. Clin. Microbiol. Rev. 17:434-464.

7. Arntzen, C., S. Plotkin, and B. Dodet. 2005. Plant-derived vaccines and antibodies: potential and limitations. Vaccine 23:1753-1756.

8. Bames, A. M., and T. J. Quan. 1992. Plague, p. 1285-1291. *In* S. Gorbach, J. Bartlett, and N. Blacklow (ed.), Infectious diseases. W. B. Saunders Company, Philadelphia, PA.

9. Benner, G. E., G. P. Andrews, W. R. Byrne, S. D. Strachan, A. K. Sample, D. G. Heath, and A. M. Friedlander. 1999. Immune response to Yersinia outer proteins and other Yersinia pestis antigens after experimental plague infection in mice. Infect. Immun. 67:1922-1928.

10. Chase, M. W. 1946. Inhibition of experimental drug allergy by prior feeding of the sensitizing agent. Proc. Exp. Biol. Med. 61:257-259.

11. Chebolu, S., and H. Daniell. 2007. Stable expression of Gal/GalNAc lectin of Entamoeba histolytica in transgenic chloroplasts and immunogenicity in mice towards vaccine development for amoebiasis. Plant Biotechnol. J. 5:230-239.

12. Collins, G. B., P. D. Legg, and M. C. Kasperbauer. 1974. Tobacco hybrid LAMD-609. Crop Sci. 14:72-80.

13. Cowan, C., A. V. Philipovskiy, C. R. Wulff-Strobel, Z. Ye, and S. C. Straley. 2005. Anti-LcrV antibody inhibits delivery of Yops by Yersinia pestis KIM5 by directly promoting phagocytosis. Infect. Immun. 73:6127-6137.

14. Daniell, H. 2002. Molecular strategies for gene containment in transgenic crops. Nat. Biotechnol. 20:581-586.

15. Daniell, H. 2006. Production of biopharmaceuticals and vaccines in plants via the chloroplast genome. Biotechnol. J. 1:1071-1079.

16. Daniell, H. 2007. Transgene containment by maternal inheritance: effective or elusive? Proc. Natl. Acad. Sci. USA 104:6879-6880.

17. Daniell, H., S. Chebolu, S. Kumar, M. Singleton, and R. Falconer. 2005. Chloroplast-derived vaccine antigens and other therapeutic proteins. Vaccine 23:1779-1783.

18. Daniell, H., S.-B. Lee, T. Panchal, and P. O. Wiebe. 2001. Expression of the native cholera toxin B subunit gene and assembly as functional oligomers in transgenic tobacco chloroplasts. J. Mol. Biol. 311:1001.

19. De Cosa, B., W. Moar, S. B. Lee, M. Miller, and H. Daniell. 2001. Overexpression of the Bt cry2Aa2 operon in chloroplasts leads to formation of insecticidal crystals. Nat. Biotechnol. 19:71-74.

20. Dhingra, A., A. R. Portis, Jr., and H. Daniell. 2004. Enhanced translation of a chloroplast-expressed RbcS gene restores small subunit levels and photosynthesis in nuclear RbcS antisense plants. Proc. Natl. Acad. Sci. USA 101:6315-6320.

21. Du, Y., R. Rosqvist, and A. Forsberg. 2002. Role of fraction 1 antigen of Yersinia pestis in inhibition of phagocytosis. Infect. Immun. 70:1453-1460.

22. Dufourmantel, N., B. Pelissier, F. Garcon, G. Peltier, J. M. Ferullo, and G. Tissot. 2004. Generation of fertile transplastomic soybean. Plant Mol. Biol 55:479-489.

23. Eyles, J. E., G. J. E. Sharp, E. D. Williamson, I. D. Spiers, and H. Oya Alpar. 1998. Intranasal administration of poly-lactic acid microsphere co-encapsulated Yersinia pestis subunits confers protection from pneumonic plague in the mouse. Vaccine 16:698-707.

24. Eyles, J. E., I. D. Spiers, E. D. Williamson, and H. Oya Alpar. 1998. Analysis of local and systemic immunological responses after intra-tracheal, intranasal and intra-muscular administration of microsphere co-encapsulated Yersinia pestis sub-unit vaccines. Vaccine 16:2000-2009.

25. Friedlander, A. M., S. L. Welkos, P. L. Worsham, G. P. Andrews, D. G. Heath, G. W. Anderson, Jr., M. L. Pitt, J. Estep, and K. Davis. 1995. Relationship between virulence and immunity as revealed in recent studies of the F1 capsule of Yersinia pestis. Clin. Infect. Dis. 21(Suppl. 2):S178-S181.

26. Galimand, M., E. Carniel, and P. Courvalin. 2006. Resistance of Yersinia pestis to antimicrobial agents. Antimicrob. Agents Chemother. 50:3233-3236.

27. Galimand, M., A. Guiyoule, G. Gerbaud, B. Rasoamanana, S. Chanteau, E. Carniel, and P. Courvalin. 1997. Multidrug resistance in Yersinia pestis mediated by a transferable plasmid. N. Engl. J. Med. 337:677-681.

28. Garmory, H. S., K. F. Griffin, K. A. Brown, and R. W. Titball. 2003. Oral immunisation with live aroA attenuated Salmonella enterica serovar Typhimurium expressing the Yersinia pestis V antigen protects mice against plague. Vaccine 21:3051-3057.

29. Glynn, A., L. C. Freytag, and J. D. Clements. 2005. Effect of homologous and heterologous prime-boost on the immune response to recombinant plague antigens. Vaccine 23:1957-1965.

30. Glynn, A., C. J. Roy, B. S. Powell, J. J. Adamovicz, L. C. Freytag, and J. D. Clements. 2005. Protection against aerosolized Yersinia pestis challenge following homologous and heterologous prime-boost with recombinant plague antigens. Infect. Immun. 73:5256-5261.

31. Greenfield, R. A., and M. S. Bronze. 2003. Prevention and treatment of bacterial diseases caused by bacterial bioterrorism threat agents. Drug Discov. Today 8:881-888.

32. Gupta, R. K., B. E. Rost, E. Relyveld, and G. R. Siber. 1995. Adjuvant properties of aluminum and calcium compounds. Pharm. Biotechnol. 6:229-248.

33. Guyton, A. C. 1947. Measurement of the respiratory volumes of laboratory animals. Am. J. Physiol. 150:70-77.

34. Hamilton, S. R., J. H. Yardley, and G. D. Brown. 1979. Suppression of local intestinal immunoglobulin A immune response to cholera toxin by subcutaneous administration of cholera toxoids. Infect. Immun. 24:422-426.

35. Heath, D. G., G. W. Anderson, J. M. Mauro, S. L. Welkos, G. P. Andrews, J. Adamovicz, and A. M. Friedlander. 1998. Protection against experimental bubonic and pneumonic plague by a recombinant capsular F1-V antigen fusion protein vaccine. Vaccine 16:1131-1137.

36. Heine, H. S., A. Louie, F. Sorgel, J. Bassett, L. Miller, L. J. Sullivan, M. Kinzig-Schippers, and G. L. Drusano. 2007. Comparison of 2 antibiotics that inhibit protein synthesis for the treatment of infection with Yersinia pestis delivered by aerosol in a mouse model of pneumonic plague. J. Infect. Dis. 196:782-787.

37. Hill, J., J. E. Eyles, S. J. Elvin, G. D. Healey, R. A. Lukaszewski, and R. W. Titball. 2006. Administration of antibody to the lung protects mice against pneumonic plague. Infect. Immun. 74:3068-3070.

38. Hill, J., S. E. Leary, K. F. Griffin, E. D. Williamson, and R. W. Titball. 1997. Regions of Yersinia pestis V antigen that contribute to protection against plague identified by passive and active immunization. Infect. Immun. 65:4476-4482.

39. Hinnebusch, B. J., M.-L. Rosso, T. G. Schwan, and E. Carniel. 2002. Highfrequency conjugative transfer of antibiotic resistance genes to Yersinia pestis in the flea midgut. Mol. Microbiol. 46:349-354.

40. Holmgren, J., and C. Czerkinsky. 2005. Mucosal immunity and vaccines. Nat. Med. 11(4 Suppl.):S45-S53.

41. Hurtle, W., L. Lindler, W. Fan, D. Shoemaker, E. Henchal, and D. Norwood. 2003. Detection and identification of ciprofloxacin-resistant Yersinia pestis by denaturing high-performance liquid chromatography. J. Clin. Microbiol. 41:3273-3283.

42. Inglesby, T. V., D. T. Dennis, D. A. Henderson, J. G. Bartlett, M. S. Ascher, E. Eitzen, A. D. Fine, A. M. Friedlander, J. Hauer, J. F. Koerner, M. Layton, J. McDade, M. T. Osterholm, T. O'Toole, G. Parker, T. M. Perl, P. K. Russell, M. Schoch-Spana, and K. Tonat. 2000. Plague as a biological weapon: medical and public health management. Working Group on Civilian Biodefense. JAMA 283:2281-2290.

43. Jones, T., J. J. Adamovicz, S. L. Cyr, C. R. Bolt, N. Bellerose, L. M. Pitt, G. H. Lowell, and D. S. Burt. 2006.

Intranasal Protollin/F1-V vaccine elicits respiratory and serum antibody responses and protects mice against lethal aerosolized plague infection. Vaccine 24:1625-1632.

44. Kamarajugadda, S., and H. Daniell. 2006. Chloroplast-derived anthrax and other vaccine antigens: their immunogenic and immunoprotective properties. Expert Rev. Vaccines 5:839-849.

45. Kanamoto, H., A. Yamashita, H. Asao, S. Okumura, H. Takase, M. Hattori, A. Yokota, and K. Tomizawa. 2006. Efficient and stable transformation of Lactuca sativa L. cv. Cisco (lettuce) plastids. Transgenic Res. 15:205-217.

46. Koster, F. T., and N. F. Pierce. 1983. Parenteral immunization causes antigen-specific cell-mediated suppression of an intestinal IgA response. J. Immunol. 131:115-119.

47. Koya, V., M. Moayeri, S. H. Leppla, and H. Daniell. 2005. Plant-based vaccine: mice immunized with chloroplast-derived anthrax protective antigen survive anthrax lethal toxin challenge. Infect. Immun. 73:8266-8274.

48. Kumar, S., and H. Daniell. 2004. Engineering the chloroplast genome for hyperexpression of human therapeutic proteins and vaccine antigens. Methods Mol. Biol. 267:365-383.

49. Kumar, S., A. Dhingra, and H. Daniell. 2004. Plastid-expressed betaine aldehyde dehydrogenase gene in carrot cultured cells, roots, and leaves confers enhanced salt tolerance. Plant Physiol. 136:2843-2854.

50. Lavelle, E. C., and D. T. O'Hagan. 2006. Delivery systems and adjuvants for oral vaccines. Expert Opin. Drug Deliv. 3:747-762.

51. Leary, S. E., E. D. Williamson, K. F. Griffin, P. Russell, S. M. Eley, and R. W. Titball. 1995. Active immunization with recombinant V antigen from Yersinia pestis protects mice against plague. Infect. Immun. 63:2854-2858.

52. Limaye, A., V. Koya, M. Samsam, and H. Daniell. 2006. Receptor-mediated oral delivery of a bioencapsulated green fluorescent protein expressed in transgenic chloroplasts into the mouse circulatory system. FASEB J. 20:959-961.

53. Lindblad, E. B. 2004. Aluminium adjuvants-in retrospect and prospect. Vaccine 22:3658-3668.

54. MacDonald, T. T. 1982. Enhancement and suppression of the Peyer's patch immune response by systemic priming. Clin. Exp. Immunol. 49:441-448.

55. Mattingly, J. A., and B. H. Waksman. 1978. Immunologic suppression after oral administration of antigen. I. Specific suppressor cells formed in rat Peyer's patches after oral administration of sheep erythrocytes and their systemic migration. J. Immunol. 121:1878-1883.

56. Mestecky, J., and J. R. McGhee. 1987. Immunoglobulin A (IgA): molecular and cellular interactions involved in IgA biosynthesis and immune response. Adv. Immunol. 40:153-245.

57. Mett, V., J. Lyons, K. Musiychuk, J. A. Chichester, T. Brasil, R. Couch, R. Sherwood, G. A. Palmer, S. J. Streatfield, and V. Yusibov. 2007. A plant-produced plague vaccine candidate confers protection to monkeys. Vaccine 25:3014-3017.

58. Meyer, K. F., J. A. Hightower, and F. R. McCrumb. 1974. Plague immunization. VI. Vaccination with the fraction I antigen of Yersinia pestis. J. Infect. Dis. 129(Suppl.):S41-S45.

59. Molina, A., S. Hervas-Stubbs, H. Daniell, A. M. Mingo-Castel, and J. Veramendi. 2004. High-yield expression of a viral peptide animal vaccine in transgenic tobacco chloroplasts. Plant Biotechnol. J. 2:141-153.

60. Morton, M., H. S. Garmory, S. D. Perkins, A. M. O'Dowd, K. F. Griffin, A. K. Turner, A. M. Bennett, and R. W. Titball. 2004. A Salmonella enterica serovar Typhi vaccine expressing Yersinia pestis F1 antigen on its surface provides protection against plague in mice. Vaccine 22:2524-2532.

61. Motin, V. L., R. Nakajima, G. B. Smirnov, and R. R. Brubaker. 1994. Passive immunity to yersiniae mediated by anti-recombinant V antigen and protein A-V antigen fusion peptide. Infect. Immun. 62:4192-4201.

62. Perry, R. D., and J. D. Fetherston. 1997. Yersinia pestis-etiologic agent of plague. Clin. Microbiol. Rev. 10:35-66.

63. Pettersson, J., A. Holmstrom, J. Hill, S. Leary, E. Frithz-Lindsten, A. von Euler-Matell, E. Carlsson, R. Titball, A. Forsberg, and H. Wolf-Watz. 1999. The V-antigen of Yersinia is surface exposed before target cell contact and involved in virulence protein translocation. Mol. Microbiol. 32:961-976.

64. Philipovskiy, A. V., C. Cowan, C. R. Wulff-Strobel, S. H. Burnett, E. J. Kerschen, D. A. Cohen, A. M. Kaplan, and S. C. Straley. 2005. Antibody against V antigen prevents Yop-dependent growth of Yersinia pestis. Infect. Immun. 73:1532-1542.

65. Pollitzer, R. 1954. Plague. WHO Monogr. Ser. 22:1-698.

66. Powell, B. S., G. P. Andrews, J. T. Enama, S. Jendrek, C. Bolt, P. Worsham, J. K. Pullen, W. Ribot, H. Hines, L. Smith, D. G. Heath, and J. J. Adamovicz. 2005. Design and testing for a nontagged F1-V fusion protein as vaccine antigen against bubonic and pneumonic plague. Biotechnol. Prog. 21:1490-1510.

67. Quesada-Vargas, T., O. N. Ruiz, and H. Daniell. 2005. Characterization of heterologous multigene operons in transgenic chloroplasts: transcription, processing, and translation. Plant Physiol. 138:1746-1762.

68. Rodrigues, C. G., C. M. Carneiro, C. T. Barbosa, and R. A. Nogueira. 1992. Antigen F1 from Yersinia pestis forms aqueous channels in lipid bilayer membranes. Braz. J. Med. Biol. Res. 25:75-79.

69. Roggenkamp, A., A. M. Geiger, L. Leitritz, A. Kessler, and J. Heesemann. 1997. Passive immunity to infection with Yersinia spp. mediated by anti-recombinant V antigen is dependent on polymorphism of V antigen. Infect. Immun. 65:446-451.

70. Ruhlman, T., R. Ahangari, A. Devine, M. Samsam, and H. Daniell. 2007. Expression of cholera toxin B-proinsulin fusion protein in lettuce and tobacco chloroplasts-oral administration protects against development of insulitis in non-obese diabetic mice. Plant Biotechnol. J. 5:495-510.

71. Ruiz, O. N., and H. Daniell. 2005. Engineering cytoplasmic male sterility via the chloroplast genome by expression of β-ketothiolase. Plant Physiol. 138:1232-1246.

72. Rydell, N., and I. Sjoholm. 2004. Oral vaccination against diphtheria using polyacryl starch microparticles as adjuvant. Vaccine 22:1265-1274.

73. Sabhnani, L., M. Manocha, K. Sridevi, D. Shashikiran, R. Rayanade, and D. N. Rao. 2003. Developing subunit immunogens using B and T cell epitopes and their constructs derived from the F1 antigen of Yersinia pestis using novel delivery vehicles. FEMS Immunol. Med. Microbiol. 38:215-229.

74. Sabhnani, L., and D. N. Rao. 2000. Identification of immunodominant epitope of F1 antigen of Yersinia pestis. FEMS Immunol. Med. Microbiol. 27:155-162.

75. Santi, L., A. Giritch, C. J. Roy, S. Marillonnet, V. Klimyuk, Y. Gleba, R. Webb, C. J. Arntzen, and H. S. Mason. 2006. Protection conferred by recombinant Yersinia pestis antigens produced by a rapid and highly scalable plant expression system. Proc. Natl. Acad. Sci. USA 103:861-866.

76. Simpson, W. J., R. E. Thomas, and T. G. Schwan. 1990. Recombinant capsular antigen (fraction 1) from Yersinia pestis induces a protective antibody response in BALB/c mice. Am. J. Trop. Med. Hyg. 43:389-396.

77. Sing, A., D. Rost, N. Tvardovskaia, A. Roggenkamp, A. Wiedemann, C. J. Kirschning, M. Aepfelbacher, and J. Heesemann. 2002. Yersinia V-antigen exploits Toll-like receptor 2 and CD14 for interleukin 10-mediated immunosuppression. J. Exp. Med. 196:1017-1024.

78. Snapper, C. M., F. D. Finkelman, and W. E. Paul. 1988. Differential regulation of IgG1 and IgE synthesis by interleukin 4. J. Exp. Med. 167:183-196.

79. Straley, S. C., and W. S. Bowmer. 1986. Virulence genes regulated at the transcriptional level by Ca2+ in Yersinia pestis include structural genes for outer membrane proteins. Infect. Immun. 51:445-454.

80. Streatfield, S. J. 2007. Approaches to achieve high-level heterologous protein production in plants. Plant Biotechnol. J. 5:2-15.

81. Taylor, V. L., R. W. Titball, and P. C. F. Oyston. 2005. Oral immunization with a dam mutant of Yersinia pseudotuberculosis protects against plague. Microbiology 151:1919-1926.

82. Titball, R. W., and E. D. Williamson. 2001. Vaccination against bubonic and pneumonic plague. Vaccine 19:4175-4184.

83. Tregoning, J. S., P. Nixon, H. Kuroda, Z. Svab, S. Clare, F. Bowe, N. Fairweather, J. Ytterberg, K. J. van Wijk, G. Dougan, and P. Maliga. 2003. Expression of tetanus toxin fragment C in tobacco chloroplasts. Nucleic Acids Res. 31:1174-1179.

84. Une, T., and R. R. Brubaker. 1984. Roles of V antigen in promoting virulence and immunity in yersiniae. J. Immunol. 133:2226-2230.

85. Verma, D., and H. Daniell. 2007. Chloroplast vector systems for biotechnology applications. Plant Physiol. 145:1129-1143.

86. Verma, D., N. P. Samson, V. Koya, and H. Daniell. 2008. A protocol for expression of foreign genes in chloroplasts. Nat. Protoc. 3:739-758.

87. Watson, J., V. Koya, S. H. Leppla, and H. Daniell. 2004. Expression of Bacillus anthracis protective antigen in transgenic chloroplasts of tobacco, a non-food/feed crop. Vaccine 22:4374-4384.

88. Weeks, S., J. Hill, A. Friedlander, and S. Welkos. 2002. Anti-V antigen antibody protects macrophages from Yersinia pestis-induced cell death and promotes phagocytosis. Microb. Pathog. 32:227-237.

89. Williams, R. C., Jr., H. Gewurz, and P. G. Quie. 1972. Effects of fraction I from Yersinia pestis on phagocytosis in vitro. J. Infect. Dis. 126:235-241.

90. Williamson, E. D. 2001. Plague vaccine research and development. J. Appl. Microbiol. 91:606-608.

91. Williamson, E. D., H. C. Flick-Smith, C. LeButt, C. A. Rowland, S. M. Jones, E. L. Waters, R. J. Gwyther, J. Miller, P. J. Packer, and M. Irving. 2005. Human immune response to a plague vaccine comprising recombinant F1 and V antigens. Infect. Immun. 73:3598-3608.

92. Williamson, E. D., H. C. Flick-Smith, E. Waters, J. Miller, I. Hodgson, C. S. Le Butt, and J. Hill. 2007. Immunogenicity of the rF1+rV vaccine for plague with identification of potential immune correlates. Microb. Pathog. 42:11-21.

93. Williamson, E. D., G. J. E. Sharp, S. M. Eley, P. M. Vesey, T. C. Pepper, R. W. Titball, and H. O. Alpar. 1996. Local and systemic immune response to a microencapsulated sub-unit vaccine for plague. Vaccine 14:1613-1619.

94. Williamson, E. D., and R. W. Titball. 2002. Vaccines against dangerous pathogens. Br. Med. Bull 62:163-173.

95. Williamson, E. D., P. M. Vesey, K. J. Gillhespy, S. M. Eley, M. Green, and R. W. Titball. 1999. An IgG1 titre to the F1 and V antigens correlates with protection against plague in the mouse model. Clin. Exp. Immunol. 116:107-114.

96. Yang, X., B. J. Hinnebusch, T. Trunkle, C. M. Bosio, Z. Suo, M. Tighe, A. Harmsen, T. Becker, K. Crist, N. Walters, R. Avci, and D. W. Pascual. 2007. Oral vaccination with Salmonella simultaneously expressing Yersinia pestis F1 and V antigens protects against bubonic and pneumonic plague. J. Immunol. 178:1059-1067.

97. Yersin, A. 1895. La peste bubonique (deuxième note). Ann. Inst. Pasteur. 9:589-592.

98. Zav'yalov, V., A. Denesyuk, G. Zav'yalova, and T. Korpela. 1995. Molecular modeling of the steric structure of the envelope F1 antigen of Yersinia pestis. Immunol. Lett. 45:19-22.

Reference to particular buffers, media, reagents, cells, culture conditions and the like, or to some subclass of same, is not intended to be limiting, but should be read to include all such related materials that one of ordinary skill in the art would recognize as being of interest or value in the particular context in which that discussion is presented. For example, it is often possible to substitute one buffer system or culture medium for another, such that a different but known way is used to achieve the same goals as those to which the use of a suggested method, material or composition is directed.

It is important to an understanding of the present invention to note that all technical and scientific terms used herein, unless defined herein, are intended to have the same meaning as commonly understood by one of ordinary skill in the art. The techniques employed herein are also those that are known to one of ordinary skill in the art, unless stated otherwise. For purposes of more clearly facilitating an understanding the invention as disclosed and claimed herein, the following definitions are provided.

## Claims

1. An orally-administrable composition comprising plant material comprising an immunogenic F1-LcrV fusion protein and *Lactuca sativa* chloroplast genomes, said chloroplast genomes comprising a Lactuca sativa pLsDV LsF1V vector, for use in a method of eliciting a protective immune response against *Y. pestis* in a subject in need thereof.

2. A Lactuca sativa plant cell homoplasmic with respect to plastids transformed to express F1 and LcrV protein, said plant cell comprising a Lactuca sativa chloroplast pLsDV LsF1V vector encoding a F1-LcrV fusion protein inserted into an intergenic region between 16S/trnI and trnA/23S genes in plastids present in said homoplasmic Lactuca sativa plant cell.

3. A method of producing a F1-LcrV fusion protein containing composition, the method including:

obtaining a stably transformed Lactuca sativa plant which includes a plastid stably transformed with an expression vector which has an expression cassette having, as operably linked components in the 5' to the 3' direction of translation, a promoter operative in a plastid operably linked to a Lactuca sativa psbA 5'UTR, a selectable marker sequence, a heterologous polynucleotide sequence coding for a F1 antigen, a LcrV antigen or a F1-LcrV fusion protein, a LcrV antigen or F1-LcrV fusion protein encoding sequence, a transcription termination sequence functional in said plastid, said expression cassette being flanked by sequences from trnI and trnA genes for homologous recombination of said expression cassette into the intergenic region between said trnI and trnA genes of said Lactuca sativa chloroplast genome; and
homogenizing material of said stably transformed Lactuca sativa plant to produce homogenized material.

4. The method of claim 3, further comprising purifying F1-V fusion protein from said homogenized material.

5. The method of claim 3, further comprising encapsulating said homogenized material.

6. The method of claim 5, wherein said homogenized material is not cooked prior to encapsulation.

7. The method of claim 3, wherein said homogenized material is dried to produce a powder.

8. The method of claim 7, further comprising encapsulating said powder.

9. The method of claim 3, wherein said lettuce expression vector comprises an expression cassette which comprises in operable linkage:

i) a 5' flanking sequence for homologous recombination with the 16S/trnI sequence in the lettuce chloroplast genome,
ii) a Prnn sequence element,
iii) a sequence encoding an aadA selectable marker,
iv) a 5'psbA UTR,
v) a sequence encoding F1-V fusion protein,
vi) a 3' psbA UTR and
v) a 3' flanking sequence for homologous recombination with the trnA/23S sequence in the lettuce chloroplast genome.

**Patentansprüche**

1. Oral verabreichbare Zusammensetzung, die pflanzliches Material umfasst, das umfasst: ein immunogenes F1-LcrV-Fusionsprotein und Lactuca-sativa-Chloroplastengenom, wobei das Chloroplastengenom umfasst: einen Lactuca sativapLsDV-LsF1V-Vektor zur Verwendung in einem Verfahren eines Hervorrufens einer schützenden Immunreaktion gegen Y. pestis bei einem davon betroffenen Patienten.

2. Lactuca-sativa-Pflanzenzelle, die bezüglich Plastiden, die transformiert sind, um F1- und LcrV-Protein zu exprimieren, homoplasmisch ist, wobei die Pflanzenzelle umfasst: einen Lactuca-sativa-Chloroplast-pLsDV-LsF1V-Vektor, der ein F1-LcrV-Fusionsprotein kodiert, der in eine intergenetische Region zwischen 16S/trnI- und trnA/23S-Genen in Plastiden eingefügt ist, die in der homoplasmischen Lactuca-sativa-Pflanzenzelle vorkommen.

3. Verfahren zum Herstellen einer Zusammensetzung, die F1-LcrV-Fusionsprotein enthält, wobei das Verfahren umfasst:

Erhalten einer stabil transformierten Lactuca-sativa-Pflanze, die umfasst: ein Plastid, das stabil mit einem Expressionsvektor transformiert ist, wobei der Expressionsvektor über eine Expressionskassette verfügt, die, als funktionsfähig verknüpfte Komponenten in der 5' zu 3'-Translationsrichtung, umfasst: einen Promoter, der in einem Plastid wirksam ist, funktionsfähig verknüpft mit einer Lactuca-sativa-psbA-5'UTR, eine auswählbare Markersequenz, eine heterologe Polynukleotidsequenz, die für ein F1-Antigen kodiert, ein LcrV-Antigen oder ein F1-LcrV-Fusionsprotein, eine LcrV-Antigen- oder F1-LcrV-Fusionsprotein-Kodiersequenz, eine Transkriptionsterminationssequenz, die in dem Plastid wirksam ist, wobei die Expressionskassette durch Sequenzen von trnl- und trnA-Genen flankiert wird, für eine homologe Rekombination der Expressionskassette in die intergenetische Region zwischen den trnl- und trnA-Genen des Lactuca-sativa-Chloroplastengenoms; und Homogenisieren von Material der stabil transformierten Lactuca-sativa-Pflanze, um homogenisiertes Material zu erzeugen.

**4.** Verfahren gemäß Anspruch 3, das weiterhin umfasst: Reinigen des F1-V-Fusionsproteins von dem homogenisierten Material.

**5.** Verfahren gemäß Anspruch 3, das weiterhin umfasst: Verkapseln des homogenisierten Materials.

**6.** Verfahren gemäß Anspruch 5, wobei das homogenisierte Material vor der Verkapselung nicht gekocht wird.

**7.** Verfahren gemäß Anspruch 3, wobei das homogenisierte Material getrocknet wird, um ein Pulver herzustellen.

**8.** Verfahren gemäß Anspruch 7, das weiterhin umfasst: Verkapseln des Pulvers.

**9.** Verfahren gemäß Anspruch 3, wobei der Salat-Expressionsvektor eine Expressionskassette umfasst, die, funktionsfähig verknüpft, umfasst:

i) eine 5'-Flankensequenz zur homologen Rekombination mit der 16S/trnI-Sequenz in dem Salat-Chloroplastengenom,
ii) ein Prnn-Sequenzelement,
iii) eine Sequenz, die einen aadA-wählbaren Marker kodiert,
iv) eine 5'psbA-UTR,
v) eine Sequenz, die F1-V-Fusionsprotein kodiert,
vi) eine 3'psbA-UTR und
v) eine 3'-Flankensequenz zur homologen Rekombination mit der trnA/23S-Sequenz in dem Salat-Chloroplastengenom.

**Revendications**

**1.** Composition administrable par voie orale comprenant un matériau végétal comprenant une protéine de fusion F1-LcrV immunogénique et des génomes de chloroplaste de *Lactuca sativa,* lesdits génomes de chloroplaste comprenant un vecteur pLsDV LsF1V de Lactuca sativa, pour une utilisation dans un procédé pour déclencher une réponse immunitaire protectrice contre *Y. pestis* chez un sujet en ayant besoin.

**2.** Cellule végétale de Lactuca sativa homoplasmique vis-à-vis de plastides transformés pour exprimer les protéines F1 et LcrV, ladite cellule végétale comprenant un vecteur pLsDV LsF1V de chloroplaste de Lactuca sativa codant une protéine de fusion F1-LcrV inséré dans une région intergénique entre les gènes 16S/*trn*I et trnA/23S dans des plastides présents dans ladite cellule végétale de Lactuca sativa homoplasmique.

**3.** Procédé de production d'une composition contenant une protéine de fusion F1-LcrV, le procédé comprenant :

l'obtention d'un plant de Lactuca sativa transformé de façon stable qui contient un plastide transformé de façon stable avec un vecteur d'expression qui a une cassette d'expression ayant, en tant que composants liés de manière fonctionnelle dans la direction de traduction de 5' vers 3', un promoteur opérationnel dans un plastide lié de manière fonctionnelle à une 5'-UTR de psbA de Lactuca sativa, une séquence de marqueur sélectionnable, une séquence polynucléotidique hétérologue codant un antigène F1, un antigène LcrV ou une protéine de fusion F1-LcrV, une séquence codant un antigène LcrV ou une protéine de fusion F1-LcrV, une séquence de terminaison de transcription fonctionnelle dans ledit plastide, ladite cassette d'expression étant flanquée par des séquences issues des gènes trnI et trnA pour une recombinaison homologue de ladite cassette d'expression dans la région intergénique entre lesdits gènes trnl et trnA dudit génome de chloroplaste de Lactuca sativa ; et l'homogénéisation du matériau dudit plant de Lactuca sativa transformé de façon stable pour produire un matériau homogénéisé.

**4.** Procédé selon la revendication 3, comprenant en outre la purification de la protéine de fusion F1-V à partir dudit matériau homogénéisé.

**5.** Procédé selon la revendication 3, comprenant en outre l'encapsulation dudit matériau homogénéisé.

**6.** Procédé selon la revendication 5, dans lequel ledit matériau homogénéisé n'est pas cuit avant l'encapsulation.

**7.** Procédé selon la revendication 3, dans lequel ledit matériau homogénéisé est séché pour produire une poudre.

**8.** Procédé selon la revendication 7, comprenant en outre l'encapsulation de ladite poudre.

**9.** Procédé selon la revendication 3, dans lequel ledit vecteur d'expression de laitue comprend une cassette d'expression qui comprend, en liaison fonctionnelle :

i) une séquence flanquante en 5' pour une recombinaison homologue avec la séquence de 16S/*trn*I dans le génome de chloroplaste de laitue,
ii) un élément de séquence de Prnn,
iii) une séquence codant un marqueur sélectionnable aadA,
iv) une UTR de psbA en 5',
v) une séquence codant une protéine de fusion F1-V,
vi) une UTR de psbA en 3', et
v) une séquence flanquante en 3' pour une recombinaison homologue avec la séquence de trnA/23S dans le génome de chloroplaste de laitue.

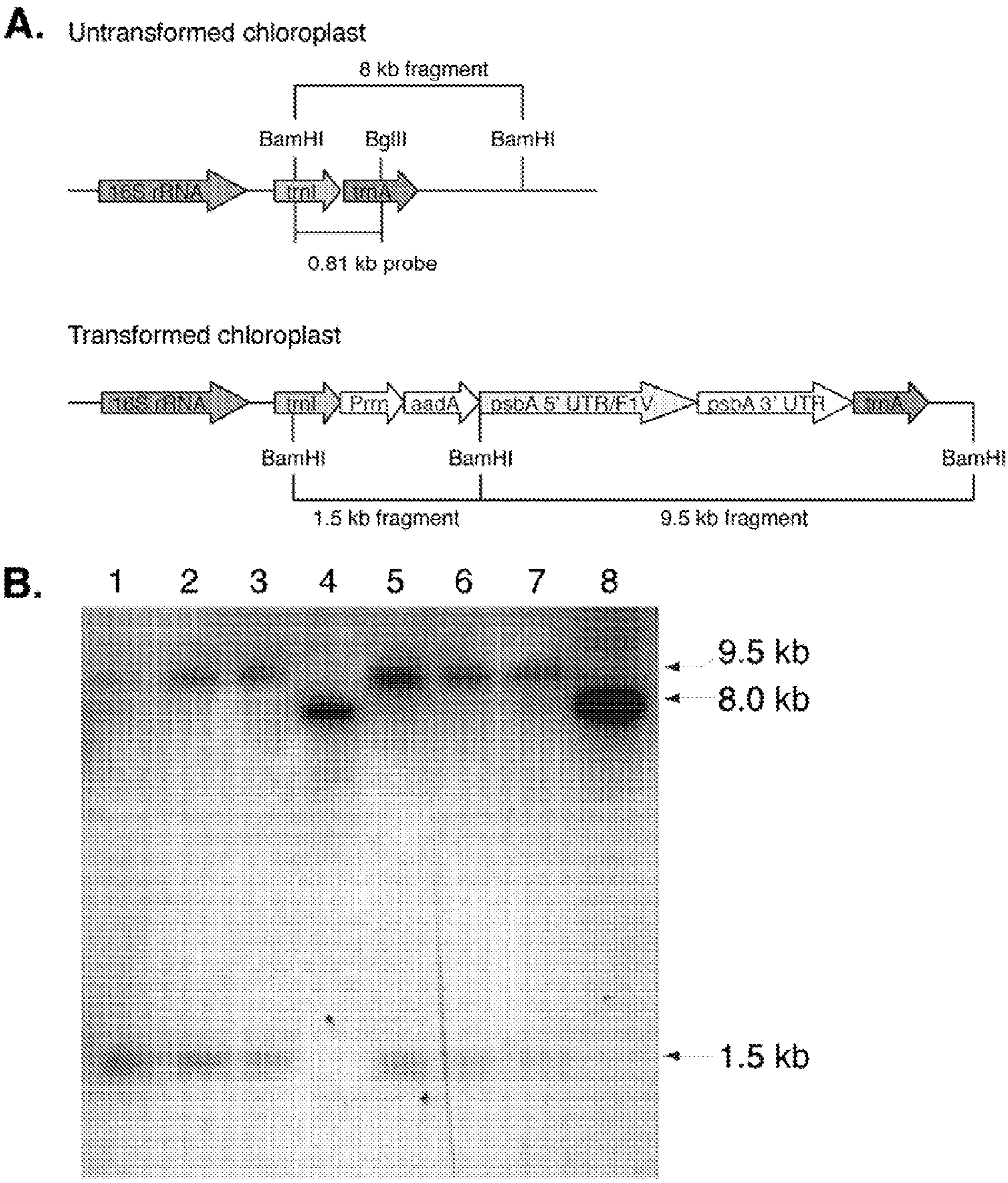
A. Untransformed chloroplast
8 kb fragment
BamHI
BglII
BamHI
16S rRNA
trnI
trnA
0.81 kb probe
Transformed chloroplast
16S rRNA
trnI
Prrn
aadA
psbA 5' UTR/F1V
psbA 3' UTR
trnA
BamHI
BamHI
BamHI
1.5 kb fragment
9.5 kb fragment
B. 1 2 3 4 5 6 7 8
9.5 kb
8.0 kb
1.5 kb

FIG. 1

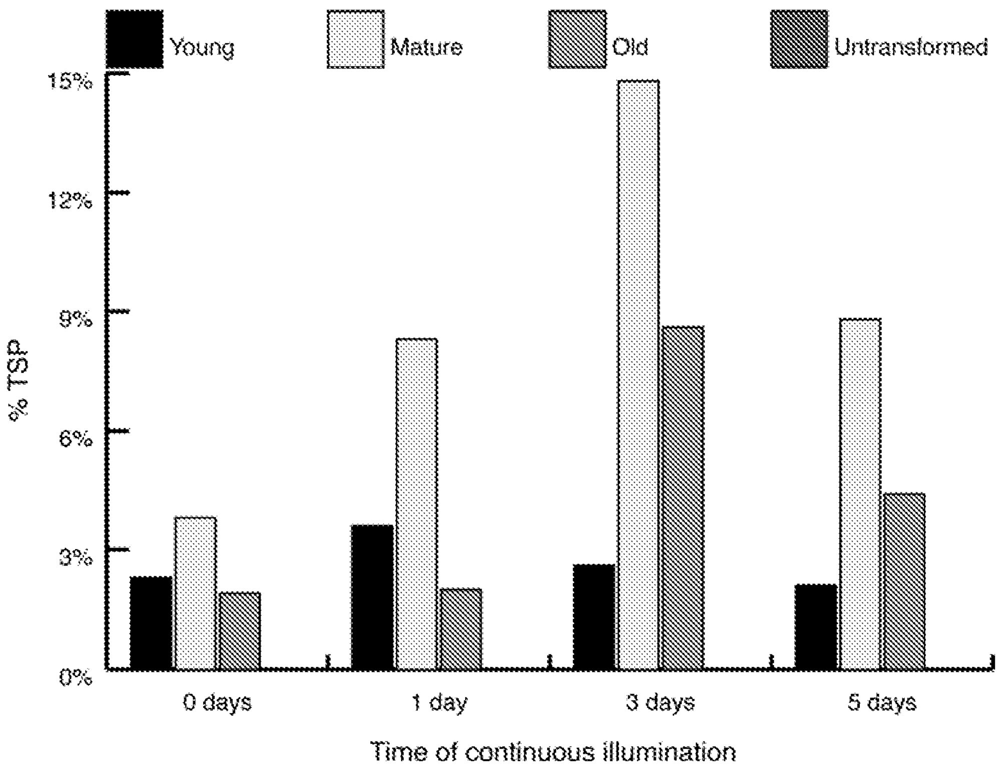
Young
Mature
Old
Untransformed
15%
12%
9%
6%
3%
0%
% TSP
0 days
1 day
3 days
5 days
Time of continuous illumination

FIG. 2

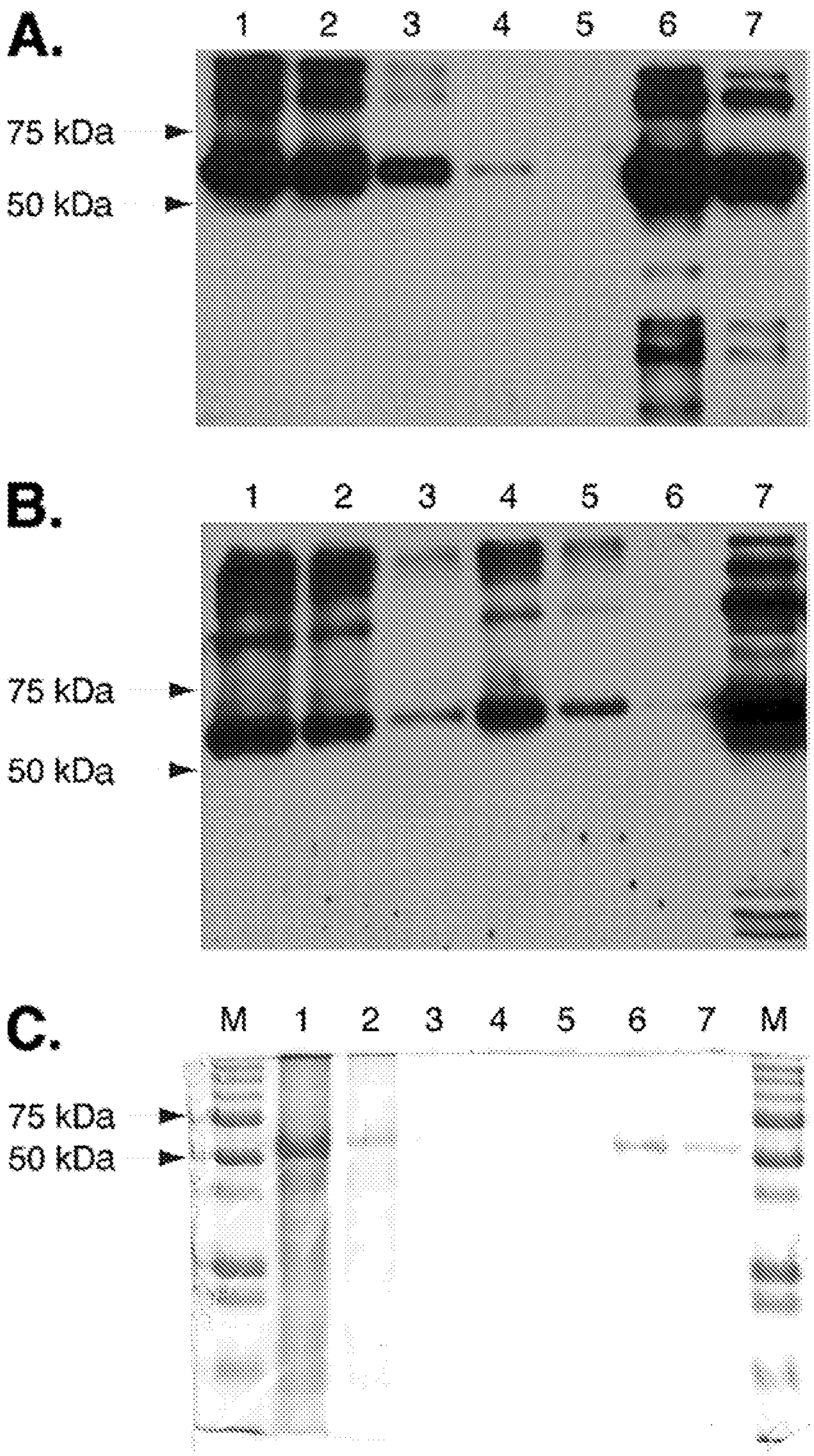
A.
1
2
3
4
5
6
7
75 kDa
50 kDa
B.
1
2
3
4
5
6
7
75 kDa
50 kDa
C.
M
1
2
3
4
5
6
7
M
75 kDa
50 kDa

FIG. 3

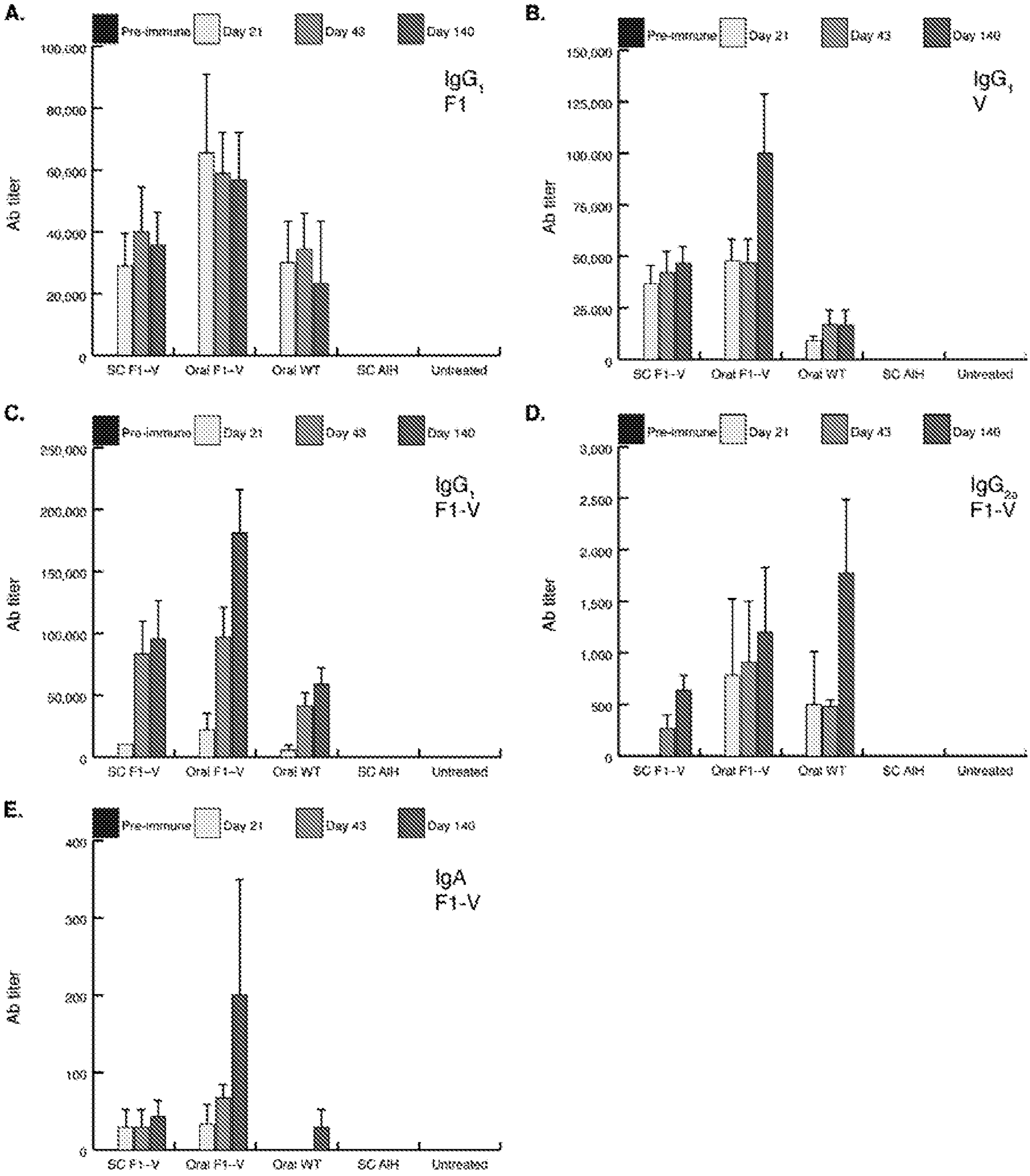
A.
Pre-immune
Day 21
Day 43
Day 140
IgG1
F1
Ab titer
SC F1-V
Oral F1-V
Oral WT
SC AlH
Untreated
B.
IgG1
V
C.
IgG1
F1-V
D.
IgG2a
F1-V
E.
IgA
F1-V

FIG. 4

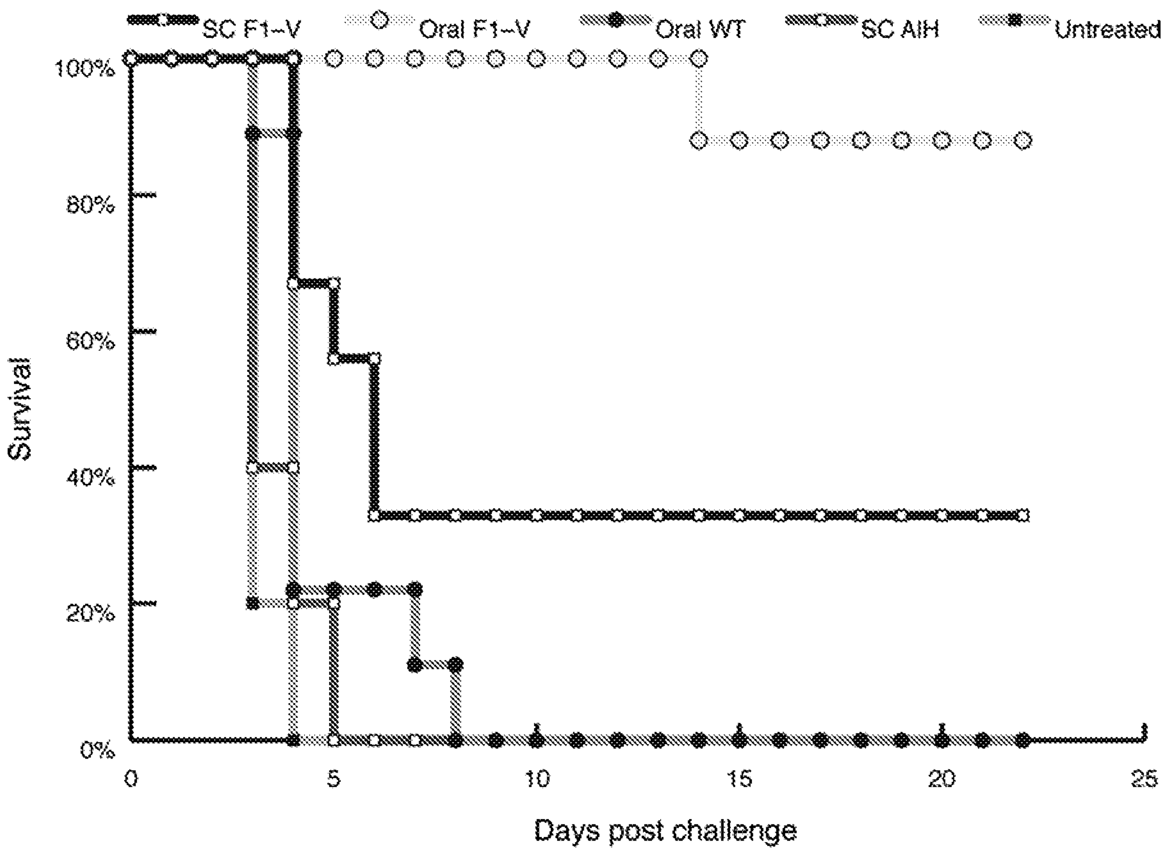
SC F1-V
Oral F1-V
Oral WT
SC AIH
Untreated
100%
80%
60%
40%
20%
0%
Survival
0
5
10
15
20
25
Days post challenge

FIG. 5

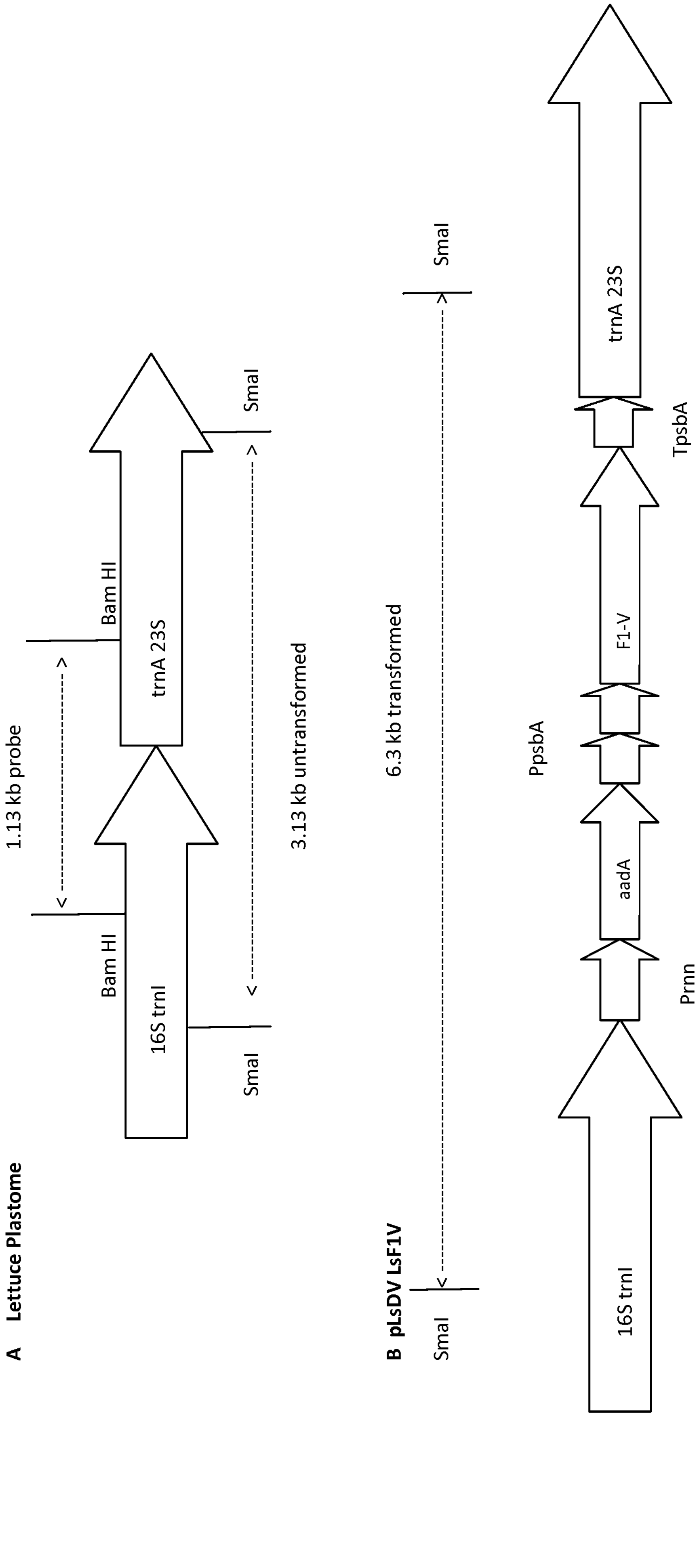
A Lettuce Plastome
1.13 kb probe
Bam HI
Bam HI
16S trnI
trnA 23S
SmaI
SmaI
3.13 kb untransformed
B pLsDV LsF1V
SmaI
6.3 kb transformed
SmaI
16S trnI
Prnn
aadA
PpsbA
F1-V
TpsbA
trnA 23S

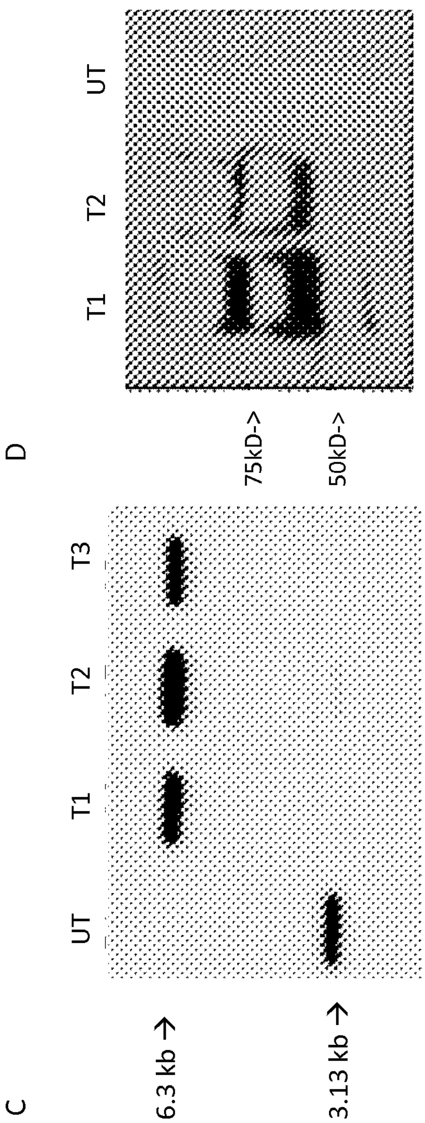
C
6.3 kb →
3.13 kb →
UT
T1
T2
T3
D
75kD->
50kD->
T1
T2
UT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61056365
- WO 0172959 A
- WO 03057834 A
- WO 04005467 A
- WO 0164023 A
- US 20090130103 A
- US 2009045215 W
- US 61056365 B

### Non-patent literature cited in the description


- **BONNER et al.** *J. Mol. Biol.,* 1973, vol. 81, 123
- **SAMBROOK et al.** MOLECULAR CLONING: A LABORATORY MANUAL. 1989, 9.50-9.51
- **BOLTON ; MCCARTHY.** *Proc. Natl. Acad. Sci. U.S.A.,* 1962, vol. 48, 1390
- **ALVAREZ, M. L. ; H. L. PINYERD ; J. D. CRISANTES ; M. M. RIGANO ; J. PINKHASOV ; A. M. WALMSLEY ; H. S. MASON ; G. A. CARDINEAU.** lant-made subunit vaccine against pneumonic and bubonic plague is orally immunogenic in mice. *Vaccine,* 2006, vol. 24, 2477-2490
- **ANDERSON, G. W., JR. ; D. G. HEATH ; C. R. BOLT ; S. L. WELKOS ; A. M. FRIEDLANDER.** Short- and long-term efficacy of single-dose subunit vaccines against Yersinia pestis in mice. *Am. J. Trop. Med. Hyg.,* 1998, vol. 58, 793-799
- **ANDERSON, G. W., JR. ; S. E. LEARY ; E. D. WILLIAMSON ; R. W. TITBALL ; S. L. WELKOS ; P. L. WORSHAM ; A. M. FRIEDLANDER.** Recombinant V antigen protects mice against pneumonic and bubonic plague caused by F1-capsule-positive and -negative strains of Yersinia pestis. *Infect. Immun.,* 1996, vol. 64, 4580-4585
- **ANDERSON, G. W., JR. ; P. L. WORSHAM ; C. R. BOLT ; G. P. ANDREWS ; S. L. WELKOS ; A. M. FRIEDLANDER ; J. P. BURANS.** Protection of mice from fatal bubonic and pneumonic plague by passive immunization with monoclonal antibodies against the F1 protein of Yersinia pestis. *Am. J. Trop. Med. Hyg.,* 1997, vol. 56, 471-473
- **ANISIMOV, A. P. ; K. K. AMOAKO.** Treatment of plague: promising alternatives to antibiotics. *J. Med. Microbiol.,* 2006, vol. 55, 1461-1475
- **ANISIMOV, A. P. ; L. E. LINDLER ; G. B. PIER.** Intraspecific diversity of Yersinia pestis. *Clin. Microbiol. Rev.,* 2004, vol. 17, 434-464
- **ARNTZEN, C. ; S. PLOTKIN ; B. DODET.** Plant-derived vaccines and antibodies: potential and limitations. *Vaccine,* 2005, vol. 23, 1753-1756
- **BAMES, A. M., ; T. J. QUAN.** *Plague,* 1992, 1285-1291
- Infectious diseases. W. B. Saunders Company
- **BENNER, G. E. ; G. P. ANDREWS ; W. R. BYRNE ; S. D. STRACHAN ; A. K. SAMPLE ; D. G. HEATH ; A. M. FRIEDLANDER.** Immune response to Yersinia outer proteins and other Yersinia pestis antigens after experimental plague infection in mice. *Infect. Immun.,* 1999, vol. 67, 1922-1928
- **CHASE, M. W.** Inhibition of experimental drug allergy by prior feeding of the sensitizing agent. *Proc. Exp. Biol. Med.,* 1946, vol. 61, 257-259
- **CHEBOLU, S. ; H. DANIELL.** Stable expression of Gal/GalNAc lectin of Entamoeba histolytica in transgenic chloroplasts and immunogenicity in mice towards vaccine development for amoebiasis. *Plant Biotechnol. J.,* 2007, vol. 5, 230-239
- **COLLINS, G. B. ; P. D. LEGG ; M. C. KASPERBAUER.** Tobacco hybrid LAMD-609. *Crop Sci.,* 1974, vol. 14, 72-80
- **COWAN, C. ; A. V. PHILIPOVSKIY ; C. R. WULFF-STROBEL ; Z. YE ; S. C. STRALEY.** Anti-LcrV antibody inhibits delivery of Yops by Yersinia pestis KIM5 by directly promoting phagocytosis. *Infect. Immun.,* 2005, vol. 73, 6127-6137
- **DANIELL, H.** Molecular strategies for gene containment in transgenic crops. *Nat. Biotechnol.,* 2002, vol. 20, 581-586
- **DANIELL, H.** Production of biopharmaceuticals and vaccines in plants via the chloroplast genome. *Biotechnol. J.,* 2006, vol. 1, 1071-1079
- **DANIELL, H.** Transgene containment by maternal inheritance: effective or elusive?. *Proc. Natl. Acad. Sci. USA,* 2007, vol. 104, 6879-6880
- **DANIELL, H. ; S. CHEBOLU ; S. KUMAR ; M. SINGLETON ; R. FALCONER.** Chloroplast-derived vaccine antigens and other therapeutic proteins. *Vaccine,* 2005, vol. 23, 1779-1783

- **DANIELL, H. ; S.-B. LEE ; T. PANCHAL ; P. O. WIEBE.** Expression of the native cholera toxin B subunit gene and assembly as functional oligomers in transgenic tobacco chloroplasts. *J. Mol. Biol.,* 2001, vol. 311, 1001
- **DE COSA, B. ; W. MOAR ; S. B. LEE ; M. MILLER ; H. DANIELL.** Overexpression of the Bt cry2Aa2 operon in chloroplasts leads to formation of insecticidal crystals. *Nat. Biotechnol.,* 2001, vol. 19, 71-74
- **DHINGRA, A. ; A. R. PORTIS, JR. ; H. DANIELL.** Enhanced translation of a chloroplast-expressed RbcS gene restores small subunit levels and photosynthesis in nuclear RbcS antisense plants. *Proc. Natl. Acad. Sci. USA,* 2004, vol. 101, 6315-6320
- **DU, Y. ; R. ROSQVIST ; A. FORSBERG.** Role of fraction 1 antigen of Yersinia pestis in inhibition of phagocytosis. *Infect. Immun.,* 2002, vol. 70, 1453-1460
- **DUFOURMANTEL, N. ; B. PELISSIER ; F. GARCON ; G. PELTIER ; J. M. FERULLO ; G. TISSOT.** Generation of fertile transplastomic soybean. *Plant Mol. Biol,* 2004, vol. 55, 479-489
- **EYLES, J. E. ; G. J. E. SHARP ; E. D. WILLIAMSON ; I. D. SPIERS ; H. OYA ALPAR.** Intranasal administration of poly-lactic acid microsphere co-encapsulated Yersinia pestis subunits confers protection from pneumonic plague in the mouse. *Vaccine,* 1998, vol. 16, 698-707
- **EYLES, J. E. ; I. D. SPIERS ; E. D. WILLIAMSON ; H. OYA ALPAR.** Analysis of local and systemic immunological responses after intra-tracheal, intranasal and intra-muscular administration of microsphere co-encapsulated Yersinia pestis sub-unit vaccines. *Vaccine,* 1998, vol. 16, 2000-2009
- **FRIEDLANDER, A. M. ; S. L. WELKOS ; P. L. WORSHAM ; G. P. ANDREWS ; D. G. HEATH ; G. W. ANDERSON, JR. ; M. L. PITT ; J. ESTEP ; K. DAVIS.** Relationship between virulence and immunity as revealed in recent studies of the F1 capsule of Yersinia pestis. *Clin. Infect. Dis.,* 1995, vol. 21 (2), S178-S181
- **GALIMAND, M. ; E. CARNIEL ; P. COURVALIN.** Resistance of Yersinia pestis to antimicrobial agents. *Antimicrob. Agents Chemother.,* 2006, vol. 50, 3233-3236
- **GALIMAND, M. ; A. GUIYOULE ; G. GERBAUD ; B. RASOAMANANA ; S. CHANTEAU ; E. CARNIEL ; P. COURVALIN.** Multidrug resistance in Yersinia pestis mediated by a transferable plasmid. *N. Engl. J. Med.,* 1997, vol. 337, 677-681
- **GARMORY, H. S. ; K. F. GRIFFIN ; K. A. BROWN ; R. W. TITBALL.** Oral immunisation with live aroA attenuated Salmonella enterica serovar Typhimurium expressing the Yersinia pestis V antigen protects mice against plague. *Vaccine,* 2003, vol. 21, 3051-3057
- **GLYNN, A. ; L. C. FREYTAG ; J. D. CLEMENTS.** Effect of homologous and heterologous prime-boost on the immune response to recombinant plague antigens. *Vaccine,* 2005, vol. 23, 1957-1965
- **GLYNN, A. ; C. J. ROY ; B. S. POWELL ; J. J. ADAMOVICZ ; L. C. FREYTAG ; J. D. CLEMENTS.** Protection against aerosolized Yersinia pestis challenge following homologous and heterologous prime-boost with recombinant plague antigens. *Infect. Immun.,* 2005, vol. 73, 5256-5261
- **GREENFIELD, R. A. ; M. S. BRONZE.** Prevention and treatment of bacterial diseases caused by bacterial bioterrorism threat agents. *Drug Discov. Today,* 2003, vol. 8, 881-888
- **GUPTA, R. K. ; B. E. ROST ; E. RELYVELD ; G. R. SIBER.** Adjuvant properties of aluminum and calcium compounds. *Pharm. Biotechnol.,* 1995, vol. 6, 229-248
- **GUYTON, A. C.** Measurement of the respiratory volumes of laboratory animals. *Am. J. Physiol.,* 1947, vol. 150, 70-77
- **HAMILTON, S. R. ; J. H. YARDLEY ; G. D. BROWN.** Suppression of local intestinal immunoglobulin A immune response to cholera toxin by subcutaneous administration of cholera toxoids. *Infect. Immun.,* 1979, vol. 24, 422-426
- **HEATH, D. G. ; G. W. ANDERSON ; J. M. MAURO ; S. L. WELKOS ; G. P. ANDREWS ; J. ADAMOVICZ ; A. M. FRIEDLANDER.** Protection against experimental bubonic and pneumonic plague by a recombinant capsular F1-V antigen fusion protein vaccine. *Vaccine,* 1998, vol. 16, 1131-1137
- **HEINE, H. S. ; A. LOUIE ; F. SORGEL ; J. BASSETT ; L. MILLER ; L. J. SULLIVAN ; M. KINZIG-SCHIPPERS ; G. L. DRUSANO.** Comparison of 2 antibiotics that inhibit protein synthesis for the treatment of infection with Yersinia pestis delivered by aerosol in a mouse model of pneumonic plague. *J. Infect. Dis.,* 2007, vol. 196, 782-787
- **HILL, J. ; J. E. EYLES ; S. J. ELVIN ; G. D. HEALEY ; R. A. LUKASZEWSKI ; R. W. TITBALL.** Administration of antibody to the lung protects mice against pneumonic plague. *Infect. Immun.,* 2006, vol. 74, 3068-3070
- **HILL, J. ; S. E. LEARY ; K. F. GRIFFIN ; E. D. WILLIAMSON ; R. W. TITBALL.** Regions of Yersinia pestis V antigen that contribute to protection against plague identified by passive and active immunization. *Infect. Immun.,* 1997, vol. 65, 4476-4482
- **HINNEBUSCH, B. J. ; M.-L. ROSSO ; T. G. SCHWAN ; E. CARNIEL.** Highfrequency conjugative transfer of antibiotic resistance genes to Yersinia pestis in the flea midgut. *Mol. Microbiol.,* 2002, vol. 46, 349-354
- **HOLMGREN, J. ; C. CZERKINSKY.** Mucosal immunity and vaccines. *Nat. Med.,* 2005, vol. 11 (4), S45-S53

- **HURTLE, W. ; L. LINDLER ; W. FAN ; D. SHOEMAKER ; E. HENCHAL ; D. NORWOOD.** Detection and identification of ciprofloxacin-resistant Yersinia pestis by denaturing high-performance liquid chromatography. *J. Clin. Microbiol.,* 2003, vol. 41, 3273-3283
- **INGLESBY, T. V. ; D. T. DENNIS ; D. A. HENDERSON ; J. G. BARTLETT ; M. S. ASCHER ; E. EITZEN ; A. D. FINE ; A. M. FRIEDLANDER ; J. HAUER ; J. F. KOERNER.** Plague as a biological weapon: medical and public health management. Working Group on Civilian Biodefense. *JAMA,* 2000, vol. 283, 2281-2290
- **JONES, T. ; J. J. ADAMOVICZ ; S. L. CYR ; C. R. BOLT ; N. BELLEROSE ; L. M. PITT ; G. H. LOWELL ; D. S. BURT.** Intranasal Protollin/F1-V vaccine elicits respiratory and serum antibody responses and protects mice against lethal aerosolized plague infection. *Vaccine,* 2006, vol. 24, 1625-1632
- **KAMARAJUGADDA, S. ; H. DANIELL.** Chloroplast-derived anthrax and other vaccine antigens: their immunogenic and immunoprotective properties. *Expert Rev. Vaccines,* 2006, vol. 5, 839-849
- **KANAMOTO, H. ; A. YAMASHITA ; H. ASAO ; S. OKUMURA ; H. TAKASE ; M. HATTORI ; A. YOKOTA ; K. TOMIZAWA.** Efficient and stable transformation of Lactuca sativa L. cv. Cisco (lettuce) plastids. *Transgenic Res.,* 2006, vol. 15, 205-217
- **KOSTER, F. T. ; N. F. PIERCE.** Parenteral immunization causes antigen-specific cell-mediated suppression of an intestinal IgA response. *J. Immunol.,* 1983, vol. 131, 115-119
- **KOYA, V. ; M. MOAYERI ; S. H. LEPPLA ; H. DANIELL.** Plant-based vaccine: mice immunized with chloroplast-derived anthrax protective antigen survive anthrax lethal toxin challenge. *Infect. Immun.,* 2005, vol. 73, 8266-8274
- **KUMAR, S. ; H. DANIELL.** Engineering the chloroplast genome for hyperexpression of human therapeutic proteins and vaccine antigens. *Methods Mol. Biol.,* 2004, vol. 267, 365-383
- **KUMAR, S. ; A. DHINGRA ; H. DANIELL.** Plastid-expressed betaine aldehyde dehydrogenase gene in carrot cultured cells, roots, and leaves confers enhanced salt tolerance. *Plant Physiol.,* 2004, vol. 136, 2843-2854
- **LAVELLE, E. C. ; D. T. O'HAGAN.** Delivery systems and adjuvants for oral vaccines. *Expert Opin. Drug Deliv.,* 2006, vol. 3, 747-762
- **LEARY, S. E. ; E. D. WILLIAMSON ; K. F. GRIFFIN ; P. RUSSELL ; S. M. ELEY ; R. W. TITBALL.** Active immunization with recombinant V antigen from Yersinia pestis protects mice against plague. *Infect. Immun.,* 1995, vol. 63, 2854-2858
- **LIMAYE, A. ; V. KOYA ; M. SAMSAM ; H. DANIELL.** Receptor-mediated oral delivery of a bioencapsulated green fluorescent protein expressed in transgenic chloroplasts into the mouse circulatory system. *FASEB J.,* 2006, vol. 20, 959-961
- **LINDBLAD, E. B.** Aluminium adjuvants-in retrospect and prospect. *Vaccine,* 2004, vol. 22, 3658-3668
- **MACDONALD, T. T.** Enhancement and suppression of the Peyer's patch immune response by systemic priming. *Clin. Exp. Immunol.,* 1982, vol. 49, 441-448
- **MATTINGLY, J. A. ; B. H. WAKSMAN.** Immunologic suppression after oral administration of antigen. I. Specific suppressor cells formed in rat Peyer's patches after oral administration of sheep erythrocytes and their systemic migration. *J. Immunol.,* 1978, vol. 121, 1878-1883
- **MESTECKY, J. ; J. R. MCGHEE.** Immunoglobulin A (IgA): molecular and cellular interactions involved in IgA biosynthesis and immune response. *Adv. Immunol.,* 1987, vol. 40, 153-245
- **METT, V. ; J. LYONS ; K. MUSIYCHUK ; J. A. CHICHESTER ; T. BRASIL ; R. COUCH ; R. SHERWOOD ; G. A. PALMER ; S. J. STREATFIELD ; V. YUSIBOV.** A plant-produced plague vaccine candidate confers protection to monkeys. *Vaccine,* 2007, vol. 25, 3014-3017
- **MEYER, K. F. ; J. A. HIGHTOWER ; F. R. MCCRUMB.** Plague immunization. VI. Vaccination with the fraction I antigen of Yersinia pestis. *J. Infect. Dis.,* 1974, vol. 129, S41-S45
- **MOLINA, A. ; S. HERVAS-STUBBS ; H. DANIELL ; A. M. MINGO-CASTEL ; J. VERAMENDI.** High-yield expression of a viral peptide animal vaccine in transgenic tobacco chloroplasts. *Plant Biotechnol. J.,* 2004, vol. 2, 141-153
- **MORTON, M. ; H. S. GARMORY ; S. D. PERKINS ; A. M. O'DOWD ; K. F. GRIFFIN ; A. K. TURNER ; A. M. BENNETT ; R. W. TITBALL.** A Salmonella enterica serovar Typhi vaccine expressing Yersinia pestis F1 antigen on its surface provides protection against plague in mice. *Vaccine,* 2004, vol. 22, 2524-2532
- **MOTIN, V. L. ; R. NAKAJIMA ; G. B. SMIRNOV ; R. R. BRUBAKER.** Passive immunity to yersiniae mediated by anti-recombinant V antigen and protein A-V antigen fusion peptide. *Infect. Immun.,* 1994, vol. 62, 4192-4201
- **PERRY, R. D. ; J. D. FETHERSTON.** Yersinia pestis-etiologic agent of plague. *Clin. Microbiol. Rev.,* 1997, vol. 10, 35-66

- **PETTERSSON, J. ; A. HOLMSTROM ; J. HILL ; S. LEARY ; E. FRITHZ-LINDSTEN ; A. VON EULER-MATELL ; E. CARLSSON ; R. TITBALL ; A. FORSBERG ; H. WOLF-WATZ.** The V-antigen of Yersinia is surface exposed before target cell contact and involved in virulence protein translocation. *Mol. Microbiol.,* 1999, vol. 32, 961-976
- **PHILIPOVSKIY, A. V. ; C. COWAN ; C. R. WULFF-STROBEL ; S. H. BURNETT ; E. J. KERSCHEN ; D. A. COHEN ; A. M. KAPLAN ; S. C. STRALEY.** Antibody against V antigen prevents Yop-dependent growth of Yersinia pestis. *Infect. Immun,* 2005, vol. 73, 1532-1542
- **POLLITZER, R.** *Plague. WHO Monogr. Ser.,* 1954, vol. 22, 1-698
- **POWELL, B. S. ; G. P. ANDREWS ; J. T. ENAMA ; S. JENDREK ; C. BOLT ; P. WORSHAM ; J. K. PULLEN ; W. RIBOT ; H. HINES ; L. SMITH.** Design and testing for a nontagged F1-V fusion protein as vaccine antigen against bubonic and pneumonic plague. *Biotechnol. Prog.,* 2005, vol. 21, 1490-1510
- **QUESADA-VARGAS, T. ; O. N. RUIZ ; H. DANIELL.** Characterization of heterologous multigene operons in transgenic chloroplasts: transcription, processing, and translation. *Plant Physiol.,* 2005, vol. 138, 1746-1762
- **RODRIGUES, C. G. ; C. M. CARNEIRO ; C. T. BARBOSA ; R. A. NOGUEIRA.** Antigen F1 from Yersinia pestis forms aqueous channels in lipid bilayer membranes. *Braz. J. Med. Biol. Res.,* 1992, vol. 25, 75-79
- **ROGGENKAMP, A. ; A. M. GEIGER ; L. LEITRITZ ; A. KESSLER ; J. HEESEMANN.** Passive immunity to infection with Yersinia spp. mediated by anti-recombinant V antigen is dependent on polymorphism of V antigen. *Infect. Immun.,* 1997, vol. 65, 446-451
- **RUHLMAN, T. ; R. AHANGARI ; A. DEVINE ; M. SAMSAM ; H. DANIELL.** Expression of cholera toxin B-proinsulin fusion protein in lettuce and tobacco chloroplasts-oral administration protects against development of insulitis in non-obese diabetic mice. *Plant Biotechnol. J.,* 2007, vol. 5, 495-510
- **RUIZ, O. N. ; H. DANIELL.** Engineering cytoplasmic male sterility via the chloroplast genome by expression of β-ketothiolase. *Plant Physiol.,* 2005, vol. 138, 1232-1246
- **RYDELL, N. ; I. SJOHOLM.** Oral vaccination against diphtheria using polyacryl starch microparticles as adjuvant. *Vaccine,* 2004, vol. 22, 1265-1274
- **SABHNANI, L. ; M. MANOCHA ; K. SRIDEVI ; D. SHASHIKIRAN ; R. RAYANADE ; D. N. RAO.** Developing subunit immunogens using B and T cell epitopes and their constructs derived from the F1 antigen of Yersinia pestis using novel delivery vehicles. *FEMS Immunol. Med. Microbiol.,* 2003, vol. 38, 215-229
- **SABHNANI, L. ; D. N. RAO.** Identification of immunodominant epitope of F1 antigen of Yersinia pestis. *FEMS Immunol. Med. Microbiol.,* 2000, vol. 27, 155-162
- **SANTI, L. ; A. GIRITCH ; C. J. ROY ; S. MARILLONNET ; V. KLIMYUK ; Y. GLEBA ; R. WEBB ; C. J. ARNTZEN ; H. S. MASON.** Protection conferred by recombinant Yersinia pestis antigens produced by a rapid and highly scalable plant expression system. *Proc. Natl. Acad. Sci. USA,* 2006, vol. 103, 861-866
- **SIMPSON, W. J. ; R. E. THOMAS ; T. G. SCHWAN.** Recombinant capsular antigen (fraction 1) from Yersinia pestis induces a protective antibody response in BALB/c mice. *Am. J. Trop. Med. Hyg.,* 1990, vol. 43, 389-396
- **SING, A. ; D. ROST ; N. TVARDOVSKAIA ; A. ROGGENKAMP ; A. WIEDEMANN ; C. J. KIRSCHNING ; M. AEPFELBACHER ; J. HEESEMANN.** Yersinia V-antigen exploits Toll-like receptor 2 and CD14 for interleukin 10-mediated immunosuppression. *J. Exp. Med.,* 2002, vol. 196, 1017-1024
- **SNAPPER, C. M. ; F. D. FINKELMAN ; W. E. PAUL.** Differential regulation of IgG1 and IgE synthesis by interleukin 4. *J. Exp. Med.,* 1988, vol. 167, 183-196
- **STRALEY, S. C. ; W. S. BOWMER.** Virulence genes regulated at the transcriptional level by Ca2+ in Yersinia pestis include structural genes for outer membrane proteins. *Infect. Immun.,* 1986, vol. 51, 445-454
- **STREATFIELD, S. J.** Approaches to achieve high-level heterologous protein production in plants. *Plant Biotechnol. J.,* 2007, vol. 5, 2-15
- **TAYLOR, V. L. ; R. W. TITBALL ; P. C. F. OYSTON.** Oral immunization with a dam mutant of Yersinia pseudotuberculosis protects against plague. *Microbiology,* 2005, vol. 151, 1919-1926
- **TITBALL, R. W. ; E. D. WILLIAMSON.** Vaccination against bubonic and pneumonic plague. *Vaccine,* 2001, vol. 19, 4175-4184
- **TREGONING, J. S. ; P. NIXON ; H. KURODA ; Z. SVAB ; S. CLARE ; F. BOWE ; N. FAIRWEATHER ; J. YTTERBERG ; K. J. VAN WIJK ; G. DOUGAN.** Expression of tetanus toxin fragment C in tobacco chloroplasts. *Nucleic Acids Res.,* 2003, vol. 31, 1174-1179
- **UNE, T. ; R. R. BRUBAKER.** Roles of V antigen in promoting virulence and immunity in yersiniae. *J. Immunol.,* 1984, vol. 133, 2226-2230
- **VERMA, D. ; H. DANIELL.** Chloroplast vector systems for biotechnology applications. *Plant Physiol.,* 2007, vol. 145, 1129-1143
- **VERMA, D. ; N. P. SAMSON ; V. KOYA ; H. DANIELL.** A protocol for expression of foreign genes in chloroplasts. *Nat. Protoc.,* 2008, vol. 3, 739-758

- **WATSON, J. ; V. KOYA ; S. H. LEPPLA ; H. DANIELL.** Expression of Bacillus anthracis protective antigen in transgenic chloroplasts of tobacco, a non-food/feed crop. *Vaccine,* 2004, vol. 22, 4374-4384
- **WEEKS, S. ; J. HILL ; A. FRIEDLANDER ; S. WELKOS.** Anti-V antigen antibody protects macrophages from Yersinia pestis-induced cell death and promotes phagocytosis. *Microb. Pathog.,* 2002, vol. 32, 227-237
- **WILLIAMS, R. C., JR. ; H. GEWURZ ; P. G. QUIE.** Effects of fraction I from Yersinia pestis on phagocytosis in vitro. *J. Infect. Dis.,* 1972, vol. 126, 235-241
- **WILLIAMSON, E. D.** Plague vaccine research and development. *J. Appl. Microbiol.,* 2001, vol. 91, 606-608
- **WILLIAMSON, E. D. ; H. C. FLICK-SMITH ; C. LEBUTT ; C. A. ROWLAND ; S. M. JONES ; E. L. WATERS ; R. J. GWYTHER ; J. MILLER ; P. J. PACKER ; M. IRVING.** Human immune response to a plague vaccine comprising recombinant F1 and V antigens. *Infect. Immun.,* 2005, vol. 73, 3598-3608
- **WILLIAMSON, E. D. ; H. C. FLICK-SMITH ; E. WATERS ; J. MILLER ; I. HODGSON ; C. S. LE BUTT ; J. HILL.** Immunogenicity of the rF1+rV vaccine for plague with identification of potential immune correlates. *Microb. Pathog.,* 2007, vol. 42, 11-21
- **WILLIAMSON, E. D. ; G. J. E. SHARP ; S. M. ELEY ; P. M. VESEY ; T. C. PEPPER ; R. W. TITBALL ; H. O. ALPAR.** Local and systemic immune response to a microencapsulated sub-unit vaccine for plague. *Vaccine,* 1996, vol. 14, 1613-1619
- **WILLIAMSON, E. D. ; R. W. TITBALL.** Vaccines against dangerous pathogens. *Br. Med. Bull,* 2002, vol. 62, 163-173
- **WILLIAMSON, E. D. ; P. M. VESEY ; K. J. GILLHESPY ; S. M. ELEY ; M. GREEN ; R. W. TITBALL.** An IgG1 titre to the F1 and V antigens correlates with protection against plague in the mouse model. *Clin. Exp. Immunol.,* 1999, vol. 116, 107-114
- **YANG, X. ; B. J. HINNEBUSCH ; T. TRUNKLE ; C. M. BOSIO ; Z. SUO ; M. TIGHE ; A. HARMSEN ; T. BECKER ; K. CRIST ; N. WALTERS.** Oral vaccination with Salmonella simultaneously expressing Yersinia pestis F1 and V antigens protects against bubonic and pneumonic plague. *J. Immunol.,* 2007, vol. 178, 1059-1067
- **YERSIN, A.** La peste bubonique (deuxième note). *Ann. Inst. Pasteur,* 1895, vol. 9, 589-592
- **ZAV'YALOV, V. ; A. DENESYUK ; G. ZAV'YALOVA ; T. KORPELA.** Molecular modeling of the steric structure of the envelope F1 antigen of Yersinia pestis. *Immunol. Lett.,* 1995, vol. 45, 19-22